# EUROPEAN PATENT SPECIFICATION

(11) **EP 4 267 821 B1**
(45) Date of publication and mention of the grant of the patent: **09.12.2026**
(21) Application number: 21908212.0
(22) Date of filing: 22.12.2021
(51) Int. Cl.: A24F 40/51, A24F 40/53, A24F 40/60, A61J 7/04, A61M 11/04, A61M 15/00, A61M 15/06, A61M 15/08, G16H 20/13

(54) **CARTRIDGE, SYSTEM AND METHOD FOR ACTIVATION OF A HEATING ASSEMBLY**
KARTUSCHE, SYSTEM UND VERFAHREN ZUR AKTIVIERUNG EINER HEIZUNGSANORDNUNG
CARTOUCHE, SYSTÈME ET PROCÉDÉ D'ACTIVATION D'UN ENSEMBLE DE CHAUFFAGE

(30) Priority: 22.12.2020 US 202063129237 P
(43) Date of publication of application: 01.11.2023
(73) Proprietor: Airgraft Inc., Montreal, Québec H3C 2N5 (CA)
(72) Inventor: BARBARIC, Mladen, Westmount, Québec H3Y 1A1 (CA); KIM, Sungmoon, Brossard, Québec J4X 2K7 (CA); LIAO, Yuhao, Mississauga, Ontario L5M 6J2 (CA); GINGRAS, Eric, Montreal, Québec H3L 1J3 (CA); WANG, Mengke, Candiac, Québec J5R 6E8 (CA); KIM, Bong Geun, Candiac, Québec J5R 0G4 (CA); CORBELLINI, Luca, Montreal, Québec H2L 1Y1 (CA); MOON, Chongchun, La Prairie, Québec J5R 0T2 (CA)
(74) Representative: Edson, Russell Gregory
(86) International application number: PCT/CA2021/051881
(87) International publication number: WO 2022/133611

(56) References cited:
- CA-A1- 3 083 248
- CA-A1- 3 086 414
- CA-A1- 3 086 414
- US-A1- 2016 331 036
- US-A1- 2017 119 058
- US-A1- 2018 263 283
- US-A1- 2020 315 253
- US-B2- 10 721 971

## Description

### Cross-Reference to Related Applications

This application claims priority- to and the benefit of U S. Provisional Application No. 63/129,237, filed December 22, 2020, entitled "Methods and Systems for Activation of a Drug Delivery Device,".

### Background

Electronic drug delivery systems are Increasingly popular. Such systems have been developed for delivery of medicinal and recreational substances. US2020315253A1 discloses utilizing data associated with a cartridge to identify an amount of aerosol to be generated by a vaporizer device, and applying to a heating element an amount of energy to achieve the desired amount of aerosol.

### Summary

According to the invention there is provided a cartridge according to claim 1, a system according to claim 2, and a method according to claim 9.

### Brief Description of the Drawings

FIG. 1 is a schematic block diagram of a system for managing an operation of a device, according to an embodiment.
FIG 2 illustrates a method of using a system, according to an embodiment.
FIG 3 illustrates a method of managing an operation of a device of a system. according to an embodiment
FIG. 4 is a schematic block diagram of a system for managing vaporizer cartridge activation, according to an embodiment.
FIG 5 illustrates a method of activating a vaporizer cartridge, according to an embodiment.
FIG. 6 is a diagram showing a cartridge configured to be coupled to a vaporizer pen, according to an embodiment.
FIG. 7 is a diagram showing the vaporizer pen of FIG. 3 configured to be coupled to the cartridge of FIG. 3, according to an embodiment.
FIG. 8 is a schematic block diagram of a system for managing container security, according to an embodiment.
FIGS. 9-13 are various views of a vaporizer device, according to an embodiment.
FIGS. 14-18 are various views of a vaporizer device, according to an embodiment.
FIGS. 19-23 are various views of a capsule dispensing system, according to an embodiment.
FIGS. 24-27 are various views of an inhaler device, according to an embodiment.
FIGS. 28-31 are various views of a nasal drug delivery device, according to an embodiment.

### Detailed Description

Systems, devices, and methods for delivery of medicinal and recreational substances are described herein. Although many of the embodiments described herein describe controlling access to medicinal and recreational substances, in some embodiments, the systems, devices and methods described herein can be used to control access to content and/or substances that are not medicinal and recreational substances.

Although the usage of medicinal and recreational substances by a user often has various benefits, users face risks associated with counterfeit products, substance abuse, and dosing non-compliance. Furthermore, many jurisdictions have implemented requirements for the manufacturing and/or usage of medicinal and recreational substances and their delivery devices. For example, with respect to electronic vapor delivery systems, various jurisdictions have implemented regulatory requirements directed toward standardization and quality control for vapor devices and associated carriers (e.g., liquids and/or solids). Moreover, counterfeit devices, cartridges, and substances in the marketplace present a safety hazard to consumers, and lead to consumer mistrust and brand dilution. As such, supply chain participants such as manufacturers and distributors are incentivized to ensure safety, authenticity and traceability of their product. For example, supply chain participants are incentivized to use systems and devices for medicinal and recreational substance delivery to a user that allow for user verification and/or identification, for validation of memberships, subscriptions, age verification, identity verification, and/or prescriptions, and for the prevention of substance abuse, dosing non-compliance, use in non-compliant physical locations (such as near schools), and use of counterfeit, recalled, or defective products. Systems and methods for achieving such objectives are set forth herein.

For example, in some embodiments, the systems and devices described herein can be operated using an activation process in which a visual identifier of a cartridge (e.g., a Quick Response (QR) code) is read by a sensor of a compute device (e.g., a mobile device or a laptop computer). The cartridge can contain any suitable contents, such as, for example, a substance to be vaporized or aerosolized, a plant material or extraction, one or more tablets, one or more capsules, or one or more pills. In response to reading the visual identifier on the cartridge, the compute device can retrieve an activation sequence (also referred to as a key indicator or a second identifier) from a command center (e.g., a cloud-based server, a centralized server and/or the like) and present the activation sequence on a display of the compute device. In some embodiments, the processor of the cartridge and/or the compute device can perform a user verification and/or membership verification step based on information stored in the command center and/or entered into or stored on the computer device prior to providing the activation sequence via the display of the compute device. A device configured to engage with the cartridge and controllably release the contents of the cartridge can interact with (e.g., read) the display of the compute device to obtain the activation sequence.

In some embodiments, the device can include, for example, a processor (also referred to as a controller) a power supply, a sensor configured to read the display of the compute device to obtain the activation sequence, and/or a mechanical and/or electrical dispensing subassembly configured to dispense or release a particular amount of the content of the cartridge under the control of the processor. The processor of the device can determine if the activation sequence obtained by the device from the compute device is based on (e.g., matches or is a transformation, derivative, or function of) a cartridge key of the cartridge and can only dispense or release the contents of the cartridge if so. In some embodiments, the processor of the device can control the dispensing subassembly to dispense or release the contents of the cartridge based on instructions included in or provided with the activation sequence (e.g., to dispense or release a particular dose).

In some embodiments, the device can include, for example, a processor (also referred to as a controller, a power supply, and/or a sensor configured to read the display of the compute device to obtain the activation sequence. The cartridge can include, for example, a processor (also referred to as a controller) and/or a mechanical and/or electrical dispensing subassembly configured to dispense or release a particular amount of the content of the cartridge under the control of the processor of the cartridge. The device can provide the activation sequence read by the sensor of the device to the processor of the cartridge. The processor of the cartridge can determine if the activation sequence obtained by the device from the compute device is based on (e.g., matches or is a transformation, derivative, or function of) a cartridge key of the cartridge and can only dispense or release the contents of the cartridge if so. In some embodiments, the processor of the cartridge can control the dispensing subassembly to dispense or release the contents of the cartridge based on instructions included in or provided with the activation sequence (e.g., to dispense or release a particular dose).

In some embodiments, an apparatus includes a housing defining an interior space, a sensor, and a processor. The sensor is configured to sense an indication generated by a compute device. The indication can be at least one of a visual indication, an auditory indication, or a vibratory indication. The processor can be configured to determine whether to transition a lock status of the apparatus from locked to unlocked based on the indication sensed by the sensor. The processor can initiate a transition of the lock status from locked to unlocked upon determining to transition the lock status.

In some embodiments, a method includes requesting initiation of an operation of a device via interaction with a user interface of a compute device. The device can be disposed adjacent a compute device such that a sensor of the device can sense an indication generated by the compute device and a processor of the device can determine whether to initiate the operation based on the indication sensed by the sensor. The indication can be at least one of a visual indication, an auditory indication, or a vibratory indication.

In some embodiments, a method includes receiving, at a processor and from a compute device, a request to allow initiation of an operation of a device, the request including at least one of an identifier of the device or an identifier of a user of the device. The processor can determine whether the request is valid based on at least one of the identifier of the device or the identifier of the user of the device. If the request is valid, an instruction can be sent to the compute device to generate an indication configured to cause the device to at least one of allow initiation of the operation or to initiate the operation. The indication can be at least one of a visual indication, an auditory indication, or a vibratory indication. If the request is not valid, the instruction to the compute device is not sent.

In some embodiments, an apparatus includes a processor and a memory operably coupled to the processor. The memory can store instructions to cause the processor to: receive, at the processor and from a compute device, a request to allow initiation of an operation of a device, the request including at least one of an identifier of the device or an identifier of a user of the device; determine, via the processor and based on at least one of the identifier of the device or the identifier of the user of the device, whether the request is valid; if the request is valid, send an instruction to the compute device to generate an indication configured to cause the device to at least one of allow initiation of the operation or to initiate the operation, the indication being at least one of a visual indication, an auditory indication, or a vibratory indication; and if the request is not valid, not send the instruction to the compute device.

In some embodiments, a system includes a vaporizer pen and a cartridge. The vaporizer pen includes a power supply and a sensor configured to sense an indication generated by a compute device. The cartridge includes a memory, a processor, and a heating assembly. The cartridge can be configured to be electrically coupled to the vaporizer pen such that the heating assembly can operate based on power provided by the power supply and such data associated with an indication sensed by the sensor can be transmitted to the processor. The processor can be configured to determine, based on the data associated with the indication sensed by the sensor, whether to allow activation of the cartridge such that the heating assembly can operate.

In some embodiments, an apparatus includes a memory storing a cartridge key, a heating assembly, a processor; and an electrical interface configured to be electrically coupled to a vaporizer pen such that the heating assembly can operate based on power received from the vaporizer pen, and such that data associated with an indication sensed by a sensor of the vaporizer pen can be transmitted to the processor via the electrical interface. The processor can be configured to determine whether the data associated with the indication includes a key indicator associated with the cartridge key, if the data associated with the indication includes the key indicator associated with the cartridge key, allow activation of the heating assembly, and if the data associated with the indication does not include the key indicator associated with the cartridge key, not allow activation of the heating assembly.

In some embodiments, a system includes a cartridge and a vaporizer pen. The cartridge can include a heating assembly. The vaporizer pen can include a power supply, a memory, a processor, and a sensor. The sensor can be configured to sense an indication generated by a compute device. The indication can be at least one of a visual indication, an auditory indication, or a vibratory indication. The vaporizer pen can be configured to be electrically coupled to the cartridge such that the heating assembly can operate based on power provided by the power supply. The processor can be configured to determine, based on data associated with the indication sensed by the sensor, whether to allow activation of the heating assembly.

In some embodiments, an apparatus includes a power supply, a memory, a processor, a sensor, and an electrical interface. The sensor can be configured to sense an indication generated by a compute device. The electrical interface is configured to be electrically coupled to a cartridge such that a heating assembly of the cartridge can operate based on power provided by the power supply. The processor can be configured to determine, based on data collected by the sensor based on the indication generated by the compute device, whether to allow activation of the heating assembly.

In some embodiments, a method includes reading, using a compute device, a cartridge identifier on an outer surface of a cartridge. The cartridge can be engaged with a vaporizer pen. The vaporizer pen can be disposed adjacent to a display of a compute device such that a sensor of the vaporizer pen can read a compute device presentation including a key indicator visually displayed on the display of the mobile device. The vaporizer pen can be configured to provide the key indicator to a processor such that the processor can determine whether or not to allow operation of a heating assembly of at least one of the cartridge or the vaporizer pen based on whether the key indicator is based on a cartridge key of the cartridge.

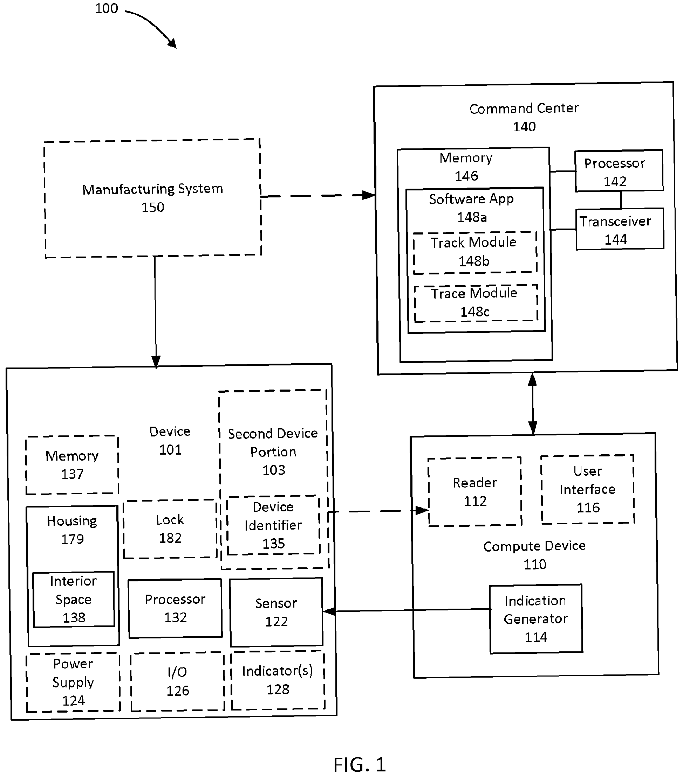

FIG. 1 is a schematic illustration of a system 100 for managing an operation of a device 101, according to some embodiments. As shown in FIG. 1, the system includes a device 101 (e.g., a consumer device or an end device) and a compute device 110. The system 100 also includes a command center 140 (e.g., a cloud-based server, a centralized server and/or the like) in wireless network communication with a compute device 110 (e.g., a mobile device such as a smartphone or tablet, a laptop, or a desktop computer of the user). The system 100 can optionally also include a manufacturing system 150 which can be in wireless network communication with the command center 140 and/or can be used to manufacture, fill, and/or package the device 101. The device 101 includes a processor 132, a sensor 122, and a housing 179. In some embodiments, the device 101 can include a memory 137. The housing 179 defines an interior space 138. The sensor 122 can sense an indication generated by the compute device 110.

The compute device 110 can include an indication generator 114 configured to present the indication (e.g., as all or a portion of a compute device presentation). In some embodiments, the indication generator 114 of the compute device 110 can include a display (e.g., liquid-crystal (LCD), organic light-emitting diode (OLED), active-matrix organic light emitting diodes (AMOLED), Super AMOLED, a thin film transistor technology (TFT) LCD, an in-place switching (IPS) LCD, a resistive touchscreen LCD, a capacitive touchscreen LCD display) configured to generate a visual or optical indication. The display can be coupled to and/or include a user interface 116 (e.g., can function as a touchscreen). In some embodiments, the indication generator 114 can be a speaker configured to generate an auditory indication. In some embodiments, the indication generator 114 can be a vibration component (e.g., a vibration motor or a piezoelectric vibrating element) configured to generate a vibratory indication. Thus, the indication can be at least one of a visual indication, an auditory indication, or a vibratory indication.

In some embodiments, a visual indication generated by the compute device 110 can include a sequence of visual images. In some embodiments, each visual image of the sequence of visual images is entirely a uniform color. In some embodiments, each visual image of the sequence of visual images is one of red, green, or blue. In some embodiments, each visual image of the sequence of visual images is one of red, green, blue, and black. In some embodiments, each visual image of the sequence of visual images is one of any suitable number of colors (e.g., two, three, four, five, six, seven, eight, nine). For example, the sequence of visual images can include six non-black colors and black. At least one visual image of the sequence of visual images can be a different color than at least one of a preceding or a subsequent visual image in the sequence. In some embodiments, in addition to or as an alternative to a sequence of images of different colors, the sequence can include a sequence or series of images having various tonal values (e.g., a sequence of greyscale images). In some embodiments, the sequence of visual images can include one or more images including a graphical arrangement. In some embodiments, the visual indication can include an image including a pattern, or a sequence of images including a pattern. In some embodiments, the visual indication can include any suitable combination of any of the images described herein. In some embodiments, each image of the visual indication displayed on the display of the compute device 110 has a larger area than the surface area of the portion of the device 101 contacting the display of the visual indication when the device 101 is properly disposed adjacent the compute device 110 for the sensing. In some embodiments, an auditory indication generated by the compute device 110 includes a sequence of sounds. Each sound in the sequence of sounds can have the same or different durations, tones, frequencies, and/or durations of non-auditory periods between sounds in the sequence of sounds. In some embodiments, a vibratory indication generated by the compute device 110 includes a sequence of vibrations. Each vibration in the sequence of vibrations can have the same or different durations, frequencies, amplitudes, and/or durations of non-vibratory periods between vibrations in the sequence of vibrations. In some embodiments, the indication can be formed by power pulses provided by the compute device 110 to the device 101 and sensed by the sensor 122.

The sensor 122 can be any suitable sensor configured to sense the indication generated by the compute device 110. For example, in some embodiments, the sensor 122 can be a light sensor (e.g., an RGB sensor) configured to sense a visual indication. In some embodiments, the sensor 122 can be an auditory sensor configured to sense an auditory indication. In some embodiments, the sensor 122 can be a vibration sensor configured to sense a vibratory indication. In some embodiments, the sensor can be coupled to an outer surface of the housing 179 and can be configured to sense the indication generated by the indication generator 114 of the compute device 110 when the device 101 (e.g., the sensor 122) is placed adjacent to the compute device 110 (e.g., when the sensor 122 is placed in contact with the compute device, such as adjacent the indication generator 114 (e.g., a display) of the computer device 110). In some embodiments, the device 101 can include a flat surface (e.g., a flat elongated side surface) that can be placed in contact with a display screen of the compute device 110. The sensor 122 can be disposed within or can be configured to sense through the flat surface or through an opening in the flat surface.

The processor 132 of the device 101 can include one or more of: a general purpose processor, a central processing unit (CPU), a microprocessor, a digital signal processor (DSP), a controller, a microcontroller, a state machine and so forth. Under some circumstances, a "processor" may refer to an application specific integrated circuit (ASIC), a programmable logic device (PLD), a field programmable gate array (FPGA), etc. The term "processor" may refer to a combination of processing devices, e.g., a combination of a DSP and a microprocessor, a plurality of microprocessors, one or more microprocessors in conjunction with a DSP core or any other such configuration. In some embodiments, the device 101 can include more than one processor 132 (e.g., a first processor associated with a first or reusable device portion of the device 101 and a second processor associated with a second device portion 103, which may be a consumable device such as a cartridge), with each processor 132 configured to perform certain tasks described herein. In some embodiments, the processor 132 can be disposed within the second device portion 103.

The memory 137 can include any electronic component capable of storing electronic information. The term memory may refer to various types of processor-readable media such as random access memory (RAM), read-only memory (ROM), non-volatile random access memory (NVRAM), programmable read-only memory (PROM), erasable programmable read only memory (EPROM), electrically erasable PROM (EEPROM), flash memory, magnetic or optical data storage, registers, etc. Memory is said to be in electronic communication with a processor if the processor can read information from and/or write information to the memory. Memory that is integral to a processor is in electronic communication with the processor. The processor 132 can be in electronic communication with the memory 137 and can be configured to read information from and/or write information to the memory 137. In some embodiments, the processor 132 and the memory 137 can be included on a common component (e.g., a common tracking component) of the device 101. The common component may be, for example, an integrated circuit (e.g., Application-Specific Integrated Circuits (ASICs)). In some embodiments, the device 101 can include more than one memory 137 (e.g., a first memory associated with a first or reusable device portion of the device 101 and a second memory associated with the second device portion 103), with each memory 137 configured to perform certain tasks described herein. In some embodiments, the memory 137 can be disposed within the second device portion 103.

The device 101 can optionally include a power supply 124 that can include any suitable battery or fuel cell, for example having high-drain characteristics. In some implementations, the device 101 can include a mechanical or electronic interface (e.g., a depressable and/or capacitive button) as part of an optional input/output (I/O) module 126 that the user can engage with to actuate an operation of the device 101. The I/O module 126 can include one or more of: a push-button control, a draw sensor (e.g., for a device 101 configured as a vaporizer device), a battery indicator, an electromechanical connector for charging and/or data communication, a light source (e.g., one or more light-emitting diodes), an interactive display, a series of buttons or dials, etc. The device 101 can also optionally include one or more indicator(s) 128 that can include one or more of: an illumination source (e.g., one or more light-emitting diodes), a speaker, a display screen (that may be the same or different from an interactive display of the input/output module 126), a vibration component (e.g., a vibration motor or a piezoelectric vibrating element), etc. In some embodiments, one or more of the indicator(s) 128 can be included in or controlled by a component of the input/output module 126. In some embodiments, the power supply 124 can be couplable to the second device portion 103 to provide operational power to the second device portion 103 (e.g., under the control of the processor 132).

The processor 132 is configured to determine whether to initiate an operation of the device 101, and/or allow initiation of an operation of the device 101 (e.g., by changing a status of the device 101) based on the indication sensed by the sensor 132. The processor 132 is also configured to initiate the operation of the device 101 and/or allow initiation of the operation of the device 101 upon determining to initiate and/or allow initiation of the operation. For example, the processor 132 can be configured to determine whether to transition a lock status of the device 101 from locked to unlocked based on the indication sensed by the sensor 132, and to initiate a transition of the lock status from locked to unlocked upon determining to transition the lock status.

In some embodiments, the device 101 can include a lock 182. The operation of the device can be a locking or unlocking operation. The lock 182 can optionally be a mechanical lock. The lock status of the device 101 can be a lock status of the mechanical lock and the mechanical lock can be in a locked configuration when the lock status is locked and the mechanical lock can be in an unlocked configuration when the lock status is unlocked. In some embodiments, the interior space 138 of the housing 179 can be inaccessible when the mechanical lock is in the locked configuration and can be accessible when the mechanical lock is in the unlocked configuration. In some embodiments, the housing 179 can include a container portion defining an opening and a lid portion couplable to the container portion to cover the opening. The container portion and the lid portion can collectively define the interior space 138 in a coupled configuration. The mechanical lock can be coupled to the lid portion, the mechanical lock configured to prevent access to an interior of the container portion through the opening in the locked configuration and to allow access to the interior of the container portion through the opening in the unlocked configuration. For example, in some embodiments, the mechanical lock can prevent the lid portion from being separated from the container portion. In some embodiments, the mechanical lock can prevent a door or tab of the lid portion from transitioning from a closed to an open configuration to allow access to or from the interior of the container portion through the lid portion. In some embodiments, the housing 179 can be a container having a door (e.g., a safe), and the lock 182 can be a mechanical lock coupled to the door and configured to prevent the door from opening relative to the housing 179 in the locked configuration and to allow the door to open relative to the housing 179 in the unlocked configuration (e.g., to provide access to the interior space 138 of the housing 179). In some embodiments, the device 101 can be couplable to a door of a room or building and can control a configuration or a position of a mechanical lock to transition the door from a locked state to an unlocked state. In such embodiments, the device 101 may not define an interior space 138.

In some embodiments, the lock 182 can be an electronic lock. The electronic lock can be associated with the device 101, a first device portion of the device 101, and/or a second device portion 103 of the device 101 (e.g., a cartridge couplable to the first device portion). In some embodiments, the processor 132 can be configured to at least one of not initiate an operation or not allow an operation to initiate (e.g., in response to interaction between a user and the I/O module 126) when a lock status of the processor 132 is "locked." The processor 132 can at least one of allow initiation of an operation or allow an operation to initiate (e.g., in response to interaction between a user and the I/O module 126) when the lock status of the processor is "unlocked." In some embodiments, the lock status is a lock status of the processor 132 with respect to a heating assembly (e.g., when the device 101 is a vaporizer pen or vaporizer device), and the heating assembly can be configured to heat contents disposed in the interior space 138 of the housing 179. For example, a heating assembly can be included in the device 101, and, if the device 101 includes a first device portion engageable with a second device portion 103 (e.g., a cartridge), the heating assembly can be included in the first device portion and/or the second device portion 103. The operation can include providing power (e.g., from the power supply 124) (e.g., to another component of the device 101 such as a heating or dispensing assembly). The operation can include heating of the heating assembly (e.g., in response to interaction between a user and the I/O module 126). In some embodiments, the lock status and operation can be associated with a dispensing operation of any suitable media (e.g., an object or a substance such as one or more pills such as tablets or capsules, one or more pellets, an aerosol substance, a vapor, a liquid substance, a spray, a powder substance, a pharmaceutical substance, and/or a controlled substance) by the device 101. For example, the device 101 can be configured to not perform the dispensing operation if the lock status of the dispensing operation, the processor 132, and/or the device 101 is locked, and to initiate the dispensing operation or allow initiation of the dispensing operation (e.g., in response to interaction between a user and the I/O module 126) in response to the lock status being changed to unlocked. In some embodiments, the dispensing operation can include dispensing of a substance from the interior space 138 of the housing 179 or a dispensing of a substance through the interior space 138 from a container coupled to the housing 179. In some embodiments, the container coupled to the housing 179 can form or be included in the second device portion 103 (e.g., a consumable device) that is couplable to a first device portion of the device 101. In some embodiments, the lock status and operation can be associated with any suitable operation of the device 101. In some embodiments, the device 101 can be or include a vaporizer, an inhaler, pill, tablet, or capsule dispensing device, a nasal delivery device, a spray device, or any other suitable delivery device.

In some embodiments, as described above, the device 101 can be configured to be coupled to or include a consumable device (e.g., as the second device portion 103) and can include or be formed as, for example, a first device portion configured as a reusable device. The first device portion can be configured to be serially coupled with multiple consumable devices for usage of each consumable device. The consumable device can be, for example, a vaporizer cartridge (also referred to as a pod or capsule), a pill dispensing cartridge, a cartridge including liquid, dry herb or plant material, or solid material for vaporization, an aerosol cartridge, or a cartridge configured to contain any other suitable object or substance and to be coupled to the device 101 or the first device portion of the device 101 such that the object(s) or substances(s) are accessible under the control of the processor 132. The device 101 can be, for example, a vaporizer pen operably couplable to the vaporizer cartridge, a dispense operably couplable to the pill dispensing cartridge, an aerosolizer operably couplable to the aerosol cartridge, a spray device operably coupled to a liquid cartridge, or any other device operably couplable to cartridge and configured to control an operation or release of an object or substance from the cartridge. In some embodiments, the second device portion 103 (e.g., a cartridge) may not include a processor, a memory, and/or any electronics, but can optionally include the identifier 135 or another identifier (e.g., a cartridge identifier). In some embodiments, as described herein, the second device portion 103 (e.g., a cartridge) can include a processor, a memory, and/or electronics (e.g., such that a cartridge key can be stored on the memory and used for analysis of a key indicator by the processor or by a processor of the first device portion of the device 101 to which the second device portion 103 is coupled). In some embodiments, the device 101 itself is disposable after delivery or dispensing of the contents of the interior space 138 (e.g., of a reservoir) and the components of the device 101 are all included within an outer housing (e.g., housing 179). In embodiments in which the device 101 is a disposable vaporizer device, for example, the outer housing and a mouthpiece of the vaporizer device can be formed such that a user cannot separate them without breaking the device (e.g., formed monolithically). In some embodiments, the first device portion (e.g., configured as a vaporizer pen) can be configured to read an identifier (e.g., identifier 135) disposed on the second device portion (e.g., the cartridge) using a sensor of the first device portion configured to read the identifier (e.g., optically) or via RF or NFC communication with the second device portion. The first device portion of the device 101 can then determine whether to operate based on the identifier on the second device portion based on any of the conditions described herein. In some embodiments, the compute device 410 can be configured to read an identifier (e.g., identifier 135) disposed on the second device portion (e.g., the cartridge) using a sensor of the compute device (e.g., the reader 412) configured to read the identifier (e.g., optically) or via RF or NFC communication with the second device portion. The first device portion of the device 101 can then determine whether to operate based on the identifier on the second device portion based on any of the conditions and method steps described herein (e.g., via communication with the compute device 410 and/or the command center 440).

In some embodiments, the device 101 will not unlock, operate, or perform one or more particular operations unless the device 101 has first been activated, validated and/or instructed (e.g., via data or instructions provided via the indication generated by the indication generator 114 of the compute device 110). The command center 140 can determine whether to send the instruction to generate the compute device presentation and the contents of the compute device presentation based on, for example, information, data, and/or requests received via the compute device 110, based on, for example, information and/or data received from the manufacturing system 150, and based on one or more conditions as described herein. In some embodiments, the command center 140 can be configured to communication with the compute device 110 and to send an instruction to the compute device 110 to generate a compute device presentation in response to a user sending a request to the command center via the user interface 116 of the compute device 116. As shown in FIG. 1, the command center 140 includes a memory 146 operably coupled to a processor 142, and a transceiver 144 configured to facilitate wireless network communications with the manufacturing system 150 and/or the compute device 110 of the user. For example, messages related to user registration and/or device 101 validation/activation (e.g., an activation or operation request message) can be exchanged between the command center 140 and the compute device 110. A request message sent to the command center 140 can be, for example, a request to unlock the device 101, for the device 101 to initiate an operation, or for the device 101 to permit an operation (e.g., controlled by the user via the I/O module 126), such as any of the operations described herein. The operation can be, for example, delivery of one or more contents of the device 101 or of a reservoir or container coupled to the device (e.g., via heating, vaporization, dispensing, and/or unlocking to make the contents accessible), or an unlocking, locking, or opening operation (e.g., to provide or restrict access of a user to an area). The request can be made, for example, using a webpage or application presented on a display of the compute device 110. In some embodiments, the request can include a device identifier 135 associated with the device 101, an identifier associated with the optional second device portion 103 of the device 101 (e.g., a cartridge) (which may be the same or different from the device identifier 135), an identifier associated with the optional first device portion of the device 101, an identifier of the user, an identifier of the compute device 110, a point-of-sale identifier (e.g., associated with a particular store or vendor), and/or any other suitable information. The command center 140 can be configured to determine whether or not to instruct the compute device 110 to generate a compute device presentation including an indication associated with activating, validating, unlocking, operating, allowing an operation, and/or instructing the device 101 (e.g., the device 101 itself, or, in cases in which the device 101 includes a first device portion and a second device portion 103, the first device portion and/or the second device portion 103) based on the request.

In some embodiments, the device identifier 135 referenced above (also referred to as a first identifier) can optionally be disposed on an outer surface of the housing 179 (which may be an outer surface of the device 101, of a first device portion of the device 101, or of a second device portion 103 of the device 101 operably couplable to the first device portion of the device 101 (e.g., a cartridge)). In some embodiments, the device identifier 135 can be a visual identifier and can include any suitable data configured to identify the device 101 (or a portion of the device 101). The device identifier 135 can be represented via any suitable visual image, such as, for example, a serial number, a barcode, and/or a QR code such that the device 101 or the portion of the device 101 may be identified and/or recognized by an external device (e.g., an assembly station and/or a fill station of the manufacturing system 150 and/or the compute device 110). In some implementations, the device identifier 135, displayed as a visual image, can be disposed on a front surface, a side surface, or a back surface of the housing 179 of the device 101. For example, the device identifier 135 can be displayed on a label affixed to an outer surface of the device 101. In some implementations, the device identifier 135 can be disposed on or included in a package containing the device 101 or the second device portion 103 (e.g., a consumable device) operably couplable to a portion of the device 101. In some implementations, the device identifier 135 can be scanned or read one or more of: during (or upon completion of) manufacturing, during (or upon completion of) filling, or when in possession of a user (e.g., scanned by a reader 112 such as a camera of the compute device 110 of the user via a software application stored thereon). The device identifier 135 can thus be used for one or more of registration, identification, validation, activation, or operational control of the device 101 (and/or a component thereof such as the second device portion 103). In some implementations, the device identifier 135 can be unique to the particular device 101 and/or a component thereof with which it is associated (i.e., each device 101 or each consumable device configured to couple to the device 101 can have a distinct device identifier 135). In some implementations, the device identifier 135 can be unique to a particular subset of devices 101 device components (e.g., consumable devices such as cartridges, containers, or capsules sharing a common characteristic such as fill contents or fill date). In some implementations, the device identifier 135 can be generated as a randomized code. In some implementations, the device identifier 135 can include or be associated with a backup code. For example, the label on device 101 or consumable couplable to the device 101 can include a device identifier 135 including both a QR code and a serial number. If the compute device 110 does not scan the QR code, or if the user prefers to input the serial number manually rather than scan the QR code, the user can input the serial number manually on a particular webpage or application of the compute device 110 to proceed with information gathering regarding the device 101 or the component of the device 101 and/or with the activation or unlocking process. In some embodiments, rather than being disposed on the device 101 or a component thereof as a visual or visible identifier, the device identifier 135 can be digitally accessible. For example, in some embodiments, the device identifier 135 can be stored on a near-field communication (NFC) chip and can be transmitted to NFC-equipped devices through an NFC transmission. In some embodiments, the device identifier 135 can be stored in a memory of the device 101 or a component thereof (e.g., the memory 137 or in a separate memory on a chip) and the device 101 or the component thereof can be Bluetooth low energy (BLE) and/or wide area network (WAN) (e.g., a cellular data network such as Long Term Evolution (LTE) or 3G) enabled for transmission of the device identifier 135 to the compute device 110.

In some embodiments, the processor 132 can be configured to be loaded with a firmware during a manufacturing phase of the processor 132 or of the device 101 such that the firmware can be programmatically used to perform authentication of the device 101 (e.g., the device 101 itself, the first device portion, and/or the second device portion 103) using one or more cryptographic methods. The processor 132 can access a key stored in the memory 137 that can be used during an authentication and/or validation process to change a lock status of the device 101 (e.g., unlock the device 101 for an operation). For example, in some implementations, the key can include a code or password that can be stored in the memory 137. The firmware of the processor 132 can include an authentication module and can be configured to access the stored key to verify that a key indicator of (e.g., included in or indicated by) the indication (also referred to as a third identifier) generated by the indication generator 114 of the compute device 110 sensed by the sensor 122 and provided to the processor 132 corresponds to the key stored in the memory 137. For example, the processor 132 can compare the key stored in the memory 137 to the key indicator of the indication generated by the indication generator 114 and received by the processor 132 and can determine whether or not to activate the device 101, initiate an operation of the device 101, or allow initiation of an operation of the device 101 (e.g., transition the lock status of the device 101 from locked to unlocked) based on whether the key indicator of the indication is based on (e.g., matches via a direct comparison, is a transformation of, is a derivative of, or corresponds to a one-way hash function of) the key stored on the memory 137. If the processor 132 determines that the key indicator of the indication is based on the key, the processor 132 can activate the device 101, initiate an operation of the device 101, or allow initiation of an operation of the device 101. If the processor 132 does not determine that the key indicator of the indication is based on the key stored in the memory 137, the device 101 will not be activated, an operation of the device 101 will not be initiated, and/or an operation of the device 101 will not be allowed to be initiated. In some implementations, the key stored in the memory 137 can include a digital signature (also referred to as an authentication signature) stored in the memory 137 (e.g., of the tracking component such as a chip) of the device 101 that can be based on a private key, and the processor 132 can determine whether to activate the device 101, initiate an operation of the device 101, or allow initiation of an operation of the device 101 based on a similar method as described with respect to authenticating and validating a tracking component in U.S. Patent Publication No. 2020/0085105 to Mladen Barbaric et al., filed on September 18, 2019 and entitled Methods and Systems for Vaporizer Security and Traceability Management and/or U.S. Patent Publication No. 2020/0113246 to Mladen Barbaric et al., filed on October 16, 2019 and entitled Variable-Viscosity Carrier Vaporizers with Enhanced Thermal and Hydrodynamic Properties.

In some embodiments, a calibration sequence can be presented in conjunction with the indication. For example, the calibration sequence and the indication can both be presented as part of a compute device presentation presented by the indication generator 114. For example, a calibration sequence can be displayed on a display of the compute device 110 prior to the display of a color, sequence of colors, and/or flashing sequence of color(s) (e.g., associated with a key indicator) of a visual indication on the display of the compute device 110. The calibration sequence can be used to ensure that the indication sensed by the sensor 122 of the device 101 is accurately understood by the processor 132 regardless of the variation or distortion in the indication generator 114 compared to other indication generators (e.g., variation in color, brightness, and/or tint of a display of the compute device 110, variation related to the speaker of the compute device 110, and/or variation related to the vibration component of the compute device 110). For example, the calibration sequence can include a predetermined sequence of colors (e.g., including each color used in the indication (e.g., in the portion of the compute device presentation associated with the key indicator)). The processor 132 can be configured to recognize the colors in the calibration sequence based on the predetermined sequence. The processor 132 can be configured to understand the indication (e.g., to identify the key indicator in the portion of the compute device presentation associated with the key indicator) based on the colors in the calibration sequence. In some embodiments, the compute device presentation can include a delimiter at the beginning and end of the sequence (e.g., prior to the calibration sequence and after the indication) such that the device 101 can identify the beginning and end of the compute device presentation. In some embodiments, the compute device presentation can be repeatedly generated by the indication generator 114 (e.g., for a duration of time or number of times) to give the device 101 sufficient opportunity to sense the compute device presentation with the sensor 122.

As discussed above, in some embodiments, the device 101 (or, in some embodiments, a portion of the device 101 such as the second device portion 103) will not unlock or operate unless the device 101 or a component thereof has first been validated and activated. In use, a user can use the compute device 110 to read the first identifier 135 disposed on the outer surface of the device 101. For example, the compute device 110 (e.g., a smartphone) can include a reader 112 (e.g., a camera and related software application stored on the compute device 110) to read the first identifier 135 (e.g., a QR code). In some embodiments, in response to reading the first identifier 135, the compute device 110 can communicate with the command center 140 to retrieve information associated with the device 101 (e.g., based on or including the data provided by the manufacturing system 150 to the command center 140 and stored in the memory 146) by sending data including the first identifier 135 to the command center 140. In response to receiving the information associated with the device 101, the compute device 110 can present an initial informative presentation (e.g., a webpage or an application) on a display of the compute device 110 (e.g., automatically) (which may be the same or different from the indication generator 114) including at least some of the information associated with the device 101. Thus, the user can view information associated with the device 101, such as a type and/or source of contents of the interior space 138 or of a reservoir or container included in the second device portion 103, concentration, dosage, and/or ingredients of the contents, etc. In some embodiments, rather than reading the first identifier 135 with the reader 112 of the compute device 110, the user can manually input a serial code associated with the first identifier 135 (e.g., printed near the QR code) and the compute device 110 can retrieve information associated with the device 101 based on the serial code.

In some embodiments, the initial informative presentation on the display of the compute device can include an interaction feature (e.g., a touch button on the screen) configured for user interaction. For example, the display can include a user interface 116 (e.g., a touchscreen). If, after reviewing the initial informative presentation, the user desires to activate, unlock, or initiate an operation of the device 101, the user can select the interaction feature (e.g., press on the button). In response to the user selecting the interaction feature, the compute device 110 can send a request message to the command center 140. The command center 140 can respond by sending instructions to the compute device 110 to present the compute device presentation on the display of the compute device 110. As described above, the compute device presentation can include an indication including, for example, a key indicator based on the key that is associated with the first identifier 135 in the memory 146 of the command center 140. Alternatively or additionally to the key indicator, the indication can also include one or more operation instructions and/or a calibration sequence as described above. The compute device presentation can include any of the indications described herein presentable by the indication generator 114. For example, in some embodiments, the compute device presentation can include a particular color or series of colors displayed over a portion or all of the display. In some embodiments, the key indicator included in the indication of the compute device presentation can be based on a conversion of the cartridge key stored as a binary code in the command center 140 to a base-6 code, for example, that can be communicated through a series of colors and/or series of flashes of one or more colors. In some embodiments, rather than the command center 140 first sending instructions to the compute device 110 to present a presentation on the display including information associated with the device 101 (e.g., the initial informative presentation) and/or sending instructions to display the compute device presentation in response to the user selecting the interaction feature, the compute device 110 can send the request message to the command center 140 in response to initially scanning the first identifier 135 and the command center 140 can send the instructions to present the compute device presentation including the key indicator to the compute device 110 with and/or instead of the instructions to present information associated with the device 101 (e.g., the initial informative presentation).

The user can arrange the device 101 against the compute device 110 (e.g., against the display of the indication generator 114) such that the sensor 122 can read the display of the compute device 110. For example, the side of the device 101 on which the sensor 122 is disposed can be placed adjacent a screen of the display such that the sensor 122 is aligned with the compute device presentation to read the color(s) and/or series of color(s) flashed and/or displayed on the display. In some embodiments, the initial informative presentation includes instructions for the user as to how to arrange the device 101 relative to the display of the compute device 110 prior to and/or during the presentation of the compute device presentation.

Upon the sensor 122 reading the compute device presentation, the processor 132 can determine (e.g., using operational power from the power supply 124) if the key indicator (e.g., code) provided via the indication of the compute device presentation is based on the key stored in the memory 137 (e.g., matches the key or is a derivative of the key). If the key indicator is confirmed to match the key stored in the memory 137, the processor 132 can transition the device 101 from a locked status to an unlocked status (e.g., activate the device 101) or perform any other suitable operation intended by the compute device presentation. Upon unlocking (e.g., activation), the device 101 can be used by the user (e.g., the contents of the interior space 138 or a reservoir or container of the second device portion 103 can be accessed). For example, in some embodiments, the processor 132 can initiate a heating assembly of the device 101 (e.g., in response to a signal from a draw sensor) using operational power from the power supply 124 of the device 101 to power the heating assembly. In some embodiments, the processor 132 can transition a mechanical lock or allow a mechanical lock to be transitioned such that an interior of the housing 179 can be accessed. Although the compute device presentation (e.g., the indication of the compute device presentation) is described above as including a key indicator, in some embodiments the compute device presentation (e.g., the indication of the compute device presentation) can include information and/or instructions for processing by the processor 132 of the device 101 in addition to or as an alternative to the key indicator.

In some embodiments, the command center 140 can provide instructions to display the compute device presentation to the compute device 110 only if the user has an active subscription status (i.e., only if the subscription status of the user has been validated). For example, upon the user reading the first identifier 135 with the reader 112 of the compute device 110, the compute device 110 can send the first identifier 135 to the command center 140 with a user identifier, an identifier of the first portion of the device 101, and/or a compute device identifier associated with the user, the device 101, and the compute device 110, respectively. The user identifier, the first device portion identifier, and/or the compute device identifier may be based on, for example, a log-in or account number assigned to the user or the compute device 110 usable to identify an account associated with the user and/or the compute device 110. The command center 140 can determine if the user identifier, the first device portion identifier, and/or the compute device identifier are associated with an active subscription. If the user identifier, the first device portion device identifier, and/or the compute device identifier is associated with an active subscription, the command center 140 can proceed with sending the instructions to the compute device 110 to generate a compute device presentation including an indication associated with activating, validating, or allowing operation of the device 101 using the indication generator 114. If the user identifier, the first device portion identifier, and/or the compute device identifier are not associated with an active subscription (e.g., a previous subscription has lapsed or a new subscription needs to be established), the command center 140 can send a communication to the compute device 110 including instructions to display a prompt for the user to subscribe or resubscribe (e.g., submit funds and/or create or update an account having a subscription). Once the user has taken the steps required to change the subscription status stored in the command center 140 from inactive to active (e.g., via engagement with the compute device 110), the compute device 110 can communicate the instructions to the compute device 110 to generate the compute device presentation including an indication associated with activating, validating, or allowing operation of the device 101. For example, the indication can include a key indicator associated with the key stored in the memory 137.

In some embodiments, the command center 140 can provide instructions to display the compute device presentation including the key indicator to the compute device 110 only if the compute device identifier corresponds to an approved, validated, or registered compute device identifier in the memory 146 of the command center 140. If the command center 140 determines that the compute device identifier is not registered, the command center 140 can transmit an instruction to the mobile device 110 to display instructions for registration of the compute device 110. In some embodiments, the command center 140 can provide instructions to display the compute device presentation including the key indicator to the compute device 110 only if the first device portion identifier or the device identifier 135 correspond to an approved, validated, or registered first device portion identifier or device identifier 135, respectively, stored in the memory 146 of the command center 140. If the command center 140 determines that the device 101 or a portion thereof is not registered, the command center 140 can transmit an instruction to the compute device 110 to display instructions for registration of the device 101 or the portion thereof. In some embodiments, the command center 140 can provide instructions to display the compute device presentation including an indication including the key indicator to the compute device 110 only if the user identifier is associated with the key (e.g., stored in the memory 137), the key indicator, and/or the device identifier 135 stored in the memory 146 of the command center 140. In some embodiments, the command center 140 can provide instructions to display the compute device presentation including the indication including the key indicator to the compute device 110 only if the cartridge activation request is sent from the compute device 110 to the command center 140 within a particular window of time (e.g., before a cutoff date or time and/or after a cutoff date or time). Thus, one or more devices 101 or components thereof can be intended for particular predetermined time periods (e.g., by a medical practitioner) and will not be able to be activated outside of the predetermined time periods. In some embodiments, a medical practitioner can transmit a prescription record or prescription update to the command center 140 to associate a prescription status and/or prescription restrictions (e.g., dosage amount and schedule) with a user identifier, a compute device identifier, a first identifier 135, and/or a first device portion identifier. In some embodiments, only a predetermined number of devices 101 or components thereof may be activated during the predetermined time period. In some embodiments, the command center 140 can implement a time-based restriction between activations of certain devices 101 or components thereof such that the activations are spaced apart to prevent over usage. In some embodiments, the command center 140 can provide instructions to display the compute device presentation including the indication including the key indicator to the compute device 110 only if the provenance record associated with the first identifier 135 can be located and verified to match the first identifier 135.

In some embodiments, the processor 132 of the device 101 can include a fraud mitigation feature to avoid a brute force activation in which a user may attempt to activate, unlock, or otherwise control the device 101 by aligning the sensor 122 (e.g., a light sensor) with an indication generator (e.g., a display) generating an indication that was not intended by the command center 140 (e.g., via a display displaying or flashing a color or series of colors (e.g., a randomized display)). The fraud mitigation feature may include a lock-out feature such that, in the event of a certain number of failed activation attempts (e.g., one, two, three) in which the sensor 122 was aligned with an indication generator but the processor 132 did not identify a match between the key stored in the memory 137 and the data or code transmitted from the indication generator via the sensor 122 (e.g., the key indicator), the processor 132 can pause or lock for a predetermined period of time (e.g., thirty seconds, two minutes, three minutes) before another activation attempt can be initiated. In some embodiments, the predetermined period of time can progressively increase with each failed activation attempt, such that the first pause of lock period is shorter than the duration of the second, which is shorter than the duration of the third, etc. For example, the first pause period can be thirty seconds, the second can be two minutes, the third can be ten minutes, the fourth can be an hour, the fifth can be six hours, etc. In some embodiments, in each instance that the processor 132 does not activate or unlock as a result of an activation attempt by the device 101 (e.g., due to a mismatch between the cartridge key and the key indicator), an activation counter of the device 101 (e.g., the processor 132) can increase an increment of the activation counter by one. If the activation counter reaches a predetermined threshold value, the device 101 or a component thereof will permanently disable itself to prevent a malicious party from attempting to gain unauthorized access to the device 101 and its contents.

In some embodiments, the memory 146 of the command center 140 can store a software application ("app") 148a. In some implementations, an administrator of the command center 140 interacts with the software app 148a via an administrator view of the app, rendered via a graphical user interface (GUI) of a compute device in wireless or wired network communication therewith, and a user interacts with the software app 148a via a user view of the app, rendered via a graphical user interface (GUI) of a compute device (e.g., a display of the compute device 110 that may be the same or separate from the indication generator 114) of the user in wireless network communication with the command center 140. The app 148a can optionally include one or more software modules, such as a track module 148b and/or a trace module 148c.

The track module 148b can include instructions to cause the processor 142 to obtain contemporaneous (e.g., real-time or substantially real-time) location information for the device 101 or a component thereof and/or one or more compute devices (e.g., the compute device 110) of a user. Such location can be obtained, for example, by querying one or more of the aforementioned devices (e.g., via one or more associated onboard location sensors of the device(s), such as a global positioning sensor (GPS) receiver). The track module 148b can also include instructions to cause the processor 142 to store the location information and, optionally, transmit the location information to one or more requestors of the wireless network (e.g., requestors associated with remote compute devices such as compute device 110 or a third party). For example, some substances (e.g., controlled substances) that may be deliverable and/or consumable via the device 101 may be lawfully consumed in certain geographical locations whereas consumption of the substances may not legally be permitted in other geographical locations (e.g., certain countries, states, counties, cities, school zones, buildings, and/or only within our outside of proximity to certain landmarks). When the reader 112 of the compute device 110 reads the device identifier 135, the track module 148b can receive (e.g., via the compute device 110 based on a geographical location of the compute device 110) contemporaneous location information associated with the device 101 or a component thereof on which the device identifier 135 is disposed. In some implementations, the track module 148b can have access to information regarding location-based permissions and/or restrictions with respect to the delivery or consumption of specific substances. Based on the location-based restriction information, the location of the device 101 or a component thereof on which the device identifier 135 is disposed, or the compute device 110, and information regarding any contents of the device 101 or a component thereof, the track module 148b can determine whether operation of the device 101 will be permitted. The processor 142 can then send instructions to the compute device 110 via the transceiver 144 based on the determination. For example, the instructions can permit the use of the device 101 by validation of the user, the device 101, and/or a component of the device 101 (e.g., by sending instructions to display the compute device presentation including the indication to the compute device 110, the indication including data configured to be understood by the processor 132 as an instruction to permit use or unlocking of the device 101), or can block or disable the use of the device 101 or the consumable device by not validating the user, the device 101, and/or the component of the device 101 (e.g., not send instructions to display the compute device presentation, send instructions to display an error message or a message including further instructions, and/or send instructions to display a compute device presentations including data (e.g., within an indication) configured to be understood by the processor 132 as an instruction not to operate, not allow operation, or not to unlock). The device 101 can then be placed sufficiently close to the compute device 110 (e.g., adjacent to the compute device 110) such that the sensor 122 can sense the compute device presentation.

In some implementations, the track module 148b can receive updated information regarding the location of the device 101 and/or a component thereof (e.g., the second device portion 103) and/or the compute device 110, and based on the updated location information, the track module 148b can update instructions related to permissions of usage. For example, based on updated location information from the reader 112 of the compute device 110 (or a different compute device 110) in a different, permissible location, the track module 148b can unblock the use of a blocked device 101 or component thereof by validating the user (e.g., via the compute device 110), the device 101, and/or the component thereof and sending instructions to the compute device 110 to generate a compute device presentation including data (e.g., within an indication) with the indication generator 114 configured to be understood by the processor 132 as an instruction to operate, allow operation, and/or unlock. The device 101 can then be placed sufficiently close to the compute device 110 (e.g., adjacent to the compute device 110) such that the sensor 122 can sense the compute device presentation.

The trace module 148c can include instructions to cause the processor 142 to request, store and/or transmit historical data associated with the manufacture and movement (e.g., within the supply chain), of one or more devices 101 and/or components thereof (e.g., the second device portion 103) and/or one or more compute devices (e.g., the compute device 110) of the user. In other words, the trace module 148c (optionally in combination with the track module 148b) monitors the chain-of-custody of one or more devices 101 or components thereof operably couplable to the device(s) 101 to ensure their safety and authenticity. The historical data can include one or more of: content, content ingredients, content formulation, provenance data, dates associated with production of the content, the device 101, and/or the consumable device, locations of manufacture and/or sale, functionality of the device 101 (e.g., in the case in which the device 101 is a vaporizer, physics of vapor generation, sequence of steps performed by the vaporizer when activated, etc.), details regarding effects of the contents within/on the user when consumed (e.g., inhaled), and/or the like. In some implementations, the historical data (or a subset thereof) is rendered via a graphical user interface (GUI) (e.g., via a display as described above with reference to the presentation) for presentation to a user, e.g., via a software application running on the compute device 110.

In some implementations, the trace module 148c can be configured to generate and maintain a list or database of devices 101 and/or components thereof (e.g., the second device portion 103). In some implementations, the trace module 148c can (optionally in combination with the track module 148b) maintain a list or database of devices 101 and/or components thereof that were authenticated at or after filling (e.g., by a filler station) and a list of device identifiers 135 associated with the authenticated devices 101 or components thereof. In some implementations, as described above, the trace module 148c can maintain a list or database of device identifiers 135 (e.g., visual identifiers or codes associated with and representable by visual identifiers) and associated key indicators (e.g., communicable via color, sound, or vibration-based identifiers includable in an indication of a compute device presentation). Each key indicator can be associated with a key (e.g., a code or electronic identifier) stored on a memory 137 of a device 101 and/or components thereof. Each device 101 and/or component thereof can be identified by the trace module 148c via the device identifiers 135. In some implementations, the trace module 148c can maintain a list or database of devices 101 and/or components thereof that can be authenticated and authorized for use (e.g., unlocked using a compute device 110) or a particular operation. For example, for devices 101 and/or components thereof authorized for use based having a device identifier 135 associated with the list or database, in response to the compute device 110 sending the device identifier 135 or data associated therewith to the command center 140, the command center 140 can provide an instruction to the compute device 110 to generate a compute device presentation including the key indicator associated with the device identifier 135 (e.g., in an indication). The compute device 110 can then generate the compute device presentation, and in response to sensing the compute device presentation with the sensor 122, the device 101 or a component thereof can determine if the key indicator included in the compute device presentation is based on the key stored in the memory 137.

In some implementations, at any point in time, a device 101, a component thereof, a set of devices 101, or components thereof, couplable to the devices 101 can be identified as being faulty or can undergo a regulatory restriction of use (e.g., restriction of use in a specific region or by a specific user group based on, for example, age). The trace module 148c can be used to generate a recall list or a block list including device identifiers 135 associated with each of the devices 101 or components thereof that have been recalled or blocked. In some embodiments, the trace module 148c can associate a recall identifier with a specific device identifier 135 (e.g., a first identifier). When an incoming request for verification, validation, or operation of a device 101 or component thereof (e.g., a second drive portion 103) is received by the command center 140 (e.g., a cartridge information or activation request including the device identifier 135) from the compute device 110, the trace module 148c can be configured to determine whether the device identifier 135 is on the recall list or has been associated with a recall flag or indication. If the device identifier 135 is determined to be on the recall list, the trace module 148c can block the validation, verification, and/or operation of the device 101 or component thereof associated with the recall. Thus, in some embodiments, in case of a recall associated with one batch of devices 101 or components thereof, the system 100 described herein can be used to block a device 101 or component thereof from being validated at a first instance of the device identifier 135 being transmitted from the compute device 110 to the command center 140 (e.g., after being read off the device 101 by the reader 112) such that the instructions to display the compute device presentation (e.g., including the key indicator) will not be provided to the compute device 110. In some embodiments, the command center 140 may provide instructions via the transceiver 144 to the compute device 110 to display information on a display of the compute device 110 associated with the recall and/or additional instructions.

In some instances, the trace module 148c can receive a request for user verification and based on a determination of a user characteristic (e.g., an age of a user), the trace module 148c can block the verification of the user for the device 101 or for a specific consumable device couplable to the device 101 associated with the device identifier 135 (e.g., by not sending instructions to display the compute device presentation including the key indicator to the compute device 110). In some implementations, the user validation or verification can include uploading an image of a government-issued identification card for review by the command center 140. For example, an image of a photo identification of the user can be included in a user identifier included in a request sent from the compute device 110 to the command center 140. The command center 140 can determine whether the photo identification is valid or invalid (e.g., based on a determination of whether the photo identification is authentic and/or unexpired). In some embodiments, the command center 140 can communicate with another command center (e.g., server), such as a third-party vendor, to determine the authenticity or validity of the photo identification in the image. If the command center 140 determines that the photo identification is invalid, the command center 140 can determine that the request is invalid. In some embodiments, the command center 140 can determine an age of the user based on the image of the photo identification. If the age is below a threshold age, which may be based on a geographic determination as described above (e.g., based on an age-based regulation associated with the geographic region) and/or on a device identifier 135 (e.g., a content included in or to be dispensed from the device 101), the command center 140 can determine that the request is invalid. In some embodiments, the command center 140 can determine if a name on the photo identification corresponds to a name stored in the memory 146 (e.g., on a list of registered or approved users), and if not, the command center 140 can determine that the request is invalid. In some embodiments, the command center 140 can determine if a name on the photo identification corresponds to a name associated with a valid prescription that has been sent to the command center 140 and/or stored in the memory 146, and if not, the command center 140 can determine that the request is invalid. In some embodiments, the user can first complete a registration step in which a registration request message is sent to the command center 140 using the compute device 110, the registration request message including an image or video of the user (e.g., taken with the reader 112 which can be a camera such as a front-facing camera of the compute device 110) and/or an image of the user's photo identification. When requesting activation, validation, or operation of the device 101, the user can submit a new image or video, also referred to as a captured image, with the request (e.g., as a user identifier of the request). The command center 140 can determine whether the captured the user of the device 101 is a registered or approved user (e.g., as stored in the memory 146) based on whether the captured image of the user corresponds to the image of a registered user (e.g., the registered user associated with a particular account, device 101, or compute device 110) stored in the memory 146. If the command center 140 determines that the person in the captured image is the same person in an earlier image submitted of the registered user (e.g., taken with a camera of the compute device 110 or at a point-of-sale terminal, such as after review of a photo identification of the user by a vendor at the point-of-sale terminal) and/or is the same person as in a submitted photo identification, the command center 140 can determine that the request is valid. Otherwise, the command center 140 can determine that the request is invalid. In some embodiments, a user may submit an image of a photo identification and one or more captured images and/or video of the user (e.g., images taken using the front-facing camera on the compute device, also referred to as "selfies") as part of one request (e.g., as different steps of completing the request on the compute device 110), and the command center 140 can evaluate whether the person whose image is on the photo identification is the same person as in the user submitted captured images and/or video. The command center 140 can also determine if the captured images and/or video were taken within a predetermined time of the request (e.g., based on associated metadata) and/or using the webpage of application associated with the command center 140. Thus, the command center 140 can determine if the attempted user of the device 101 is the same person as pictured on a submitted photo identification and only determine that the request is valid if the attempted user of the device 101 (shown in the captured images and/or video) is a match for the person shown on the photo identification. In some implementations, the trace module 148c can be configured to implement a substance block for a particular user based on the user verification such that particular content or substances dispensable, accessible, or vaporizable by the device 101 (e.g., associated with particular content identifier(s)) can be validated for use by the user and other content or substances cannot be validated (e.g., based on regulatory age restrictions). Upon validating or verifying the user, the command center 140 can send the instruction to display the compute device presentation (e.g., including the key indicator) to the compute device 110.

In some embodiments, the command center 140 and/or the compute device 110 can detect a fraudulent attempt to use an image or video on another compute device, screen, display, or other medium to pass the likeness check. For example, the command center 140 can determine that the captured image, and thus the request, is invalid based on a screen frequency or pixel detection metric of the captured image. Thus, if a user who is not the registered or approved user of the device 101 attempts to capture an image or video using a camera of the compute device 110 of the registered or approved user associated with the device 101 and/or the compute device 110 displayed on another compute device (e.g., on a display of a mobile phone, tablet, or laptop), the command center 140 can recognize that the captured image is an image of an image from a display of another compute device and determine that the request as invalid (e.g., reject the request).

To prevent a user from validating or unlocking the device 101 and then transferring possession of the device 101 to another user (e.g., a user who would not have been approved by the command center 140 due to lacking any of the characteristics described herein such as a particular age or prescription status), in some embodiments, the command center 140 can be configured to request likeness checks at preset or random intervals or at key events (e.g., initial registration, activation of a new device, reloading an account balance). For example, in some embodiments, the command center 140 can be configured to include a time-based or usage-based limit associated with the operation of the device 101 in the instructions sent to the compute device 110 regarding the presentation of the compute device presentation. The command center 140 can send instructions to generate a compute device presentation including a period of time (e.g., since last validation or total usage time such as draw time on a vaporizer device), a usage counter threshold, or a dosage threshold until the device 101 and/or the user will need to be re-validated to continue use of the device 101. Upon the device 101 reaching the end of the period of time, the usage counter threshold, or the dosage threshold, the device 101 will stop operating (e.g., will lock). To continue usage of the device 101, the user will need to send a new request using the compute device 110 to the command center. In some embodiments, the user will be required to capture new image(s) or video to include in the request (e.g., as a user identifier). The command center 140 can determine whether the user in the newly captured images is the same user as in a photo identification (e.g., a photo identification submitted during registration) or in a previously captured and stored image(s) or video of the user, and will only validate the request (and send instructions to the compute device 110 to generate a compute device presentation including an indication associated with validating or unlocking the device 101) upon determining that the user in the captured images is the same user in the photo identification or previously captured image(s) or video. In some embodiments, alternatively or in addition to the command center 140 including an instruction for the device 101 to cease operation after a particular usage or time-based limit being met, the command center 140 can send an instruction to the compute device 110 to request a likeness check (e.g., a new image or video of the user to be captured for comparison with an image stored in the memory 146). In response, the compute device 110 can present an alert for the user (e.g., a push alert). In some embodiments, the compute device 110 can communicate with the device 101 to suspend operation of the device 101 (e.g., via a Bluetooth communication, an NFC communication, a text message initiated by a WAN connection, or via the indication generator 114 generating a compute device presentation including an instruction to suspend operation the next time that the device 101 is placed in contact with the compute device 110 to sense the indication generated from the indication generator 114). After the user has satisfied the request of the command center 140 by submitting a newly captured image or video and the command center 140 has determined that the subject of the captured image or video matches an image of the user in the memory 146, the command center 140 can send an instruction to the compute device 110 to present a compute device presentation including an indication such that the device 101 can resume operation after sensing the indication.

In some embodiments, the image or video of the user can be two-dimensional. In some embodiments, the image or video can be three-dimensional and/or a user's face can be mapped using three-dimensional mapping. In some embodiments, as an alternative or in addition to performing a likeness check using a captured image or video of the user, the system 100 can be configured to sense or collect other biometric or personal data from the user to be compared to biometric or personal data stored in the memory 146 as part of a determination of whether to allow the device 101 to operate or unlock. For example, in some embodiments, the compute device 110 can include a fingerprint scanner and/or a retinal scanner such that fingerprint and/or retina data can be collected and compared to fingerprint and/or retina data stored in the memory 146 (e.g., at the time of purchase and/or registration of a device 101). In some embodiments, a sensor such as a pulse oximeter or a camera and flash of a mobile device configured to read pulse waves of a user's finger to detect a pulse wave signature of the user. The pulse wave signature can be compared to a pulse wave signature stored in the memory 146 to determine whether the pulse wave signature matches a pulse wave signature stored in the memory 146 (e.g., at the time of purchase and/or registration of a device 101). In some embodiments, a device 101 can be associated with more than one user (e.g., in the memory 146) such that each user's images, biometric data, or personal data can be used to unlock or operate the device 101. Thus, users sharing a household can both use the device 101. In some embodiments, voice recognition can be used to identify if a user is the same user as previously registered or validated by the command center 140. In some embodiments, the device 101 rather than or in addition to the compute device 110 can include a sensor configured to collect the biometric or personal data (e.g., a microphone, camera, fingerprint sensor). The processor 132 of device 101 can be configured to compare the collected data to stored identity data on the device 101, or the device 101 can provide the collected data to the compute device 110 or the command center 140 (e.g., via the compute device 110) for comparison analysis.

In some embodiments, to prevent unwanted usage of the device 101, the user can use the compute device 110 to lock the device 101 for a period of time or until the user uses the compute device 110 to unlock the device 101. For example, the user can interact with a webpage or app associated with the device 101 and/or the command center 140 using the user interface of the compute device 110, and request that the device 101 be locked. In some embodiments, the compute device 110 can generate a compute device presentation using the indication generator 114 (e.g., including a visual, auditory, or vibratory sequence as described above) including instructions for the device 101 to lock. The device 101 can then be placed adjacent the compute device 110 to sense the compute device presentation. The processor 132 can then lock in response to the sensed instructions.

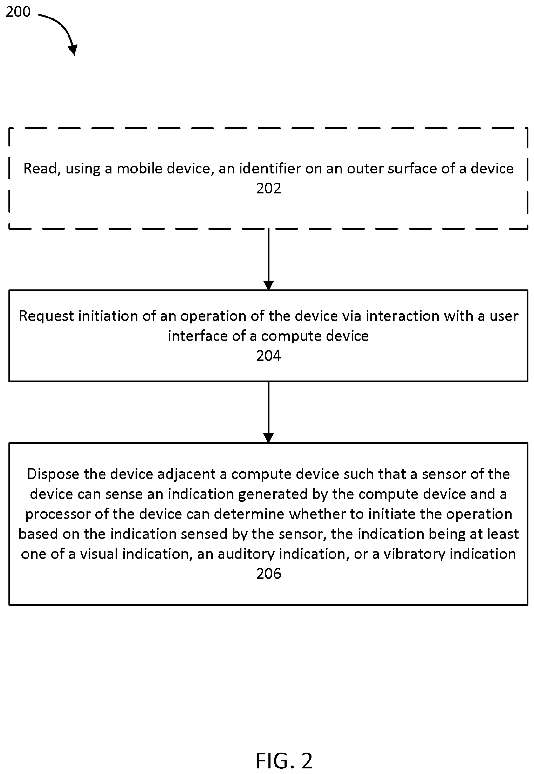

FIG. 2 illustrates a method 200 of using any of the systems described herein, such as the system 100 described above. The method 200 includes reading, at 202, an identifier (e.g., a device identifier such as device identifier 135 described above) on an outer surface of a device using a mobile device. At 204, initiation of an operation of the device can be requested via interaction with a user interface of a compute device. At 206, the device can be disposed adjacent a compute device such that a sensor of the device can sense an indication generated by the compute device and a processor of the device can determine whether to initiate the operation based on the indication sensed by the sensor. The indication can be at least one of a visual indication, an auditory indication, or a vibratory indication.

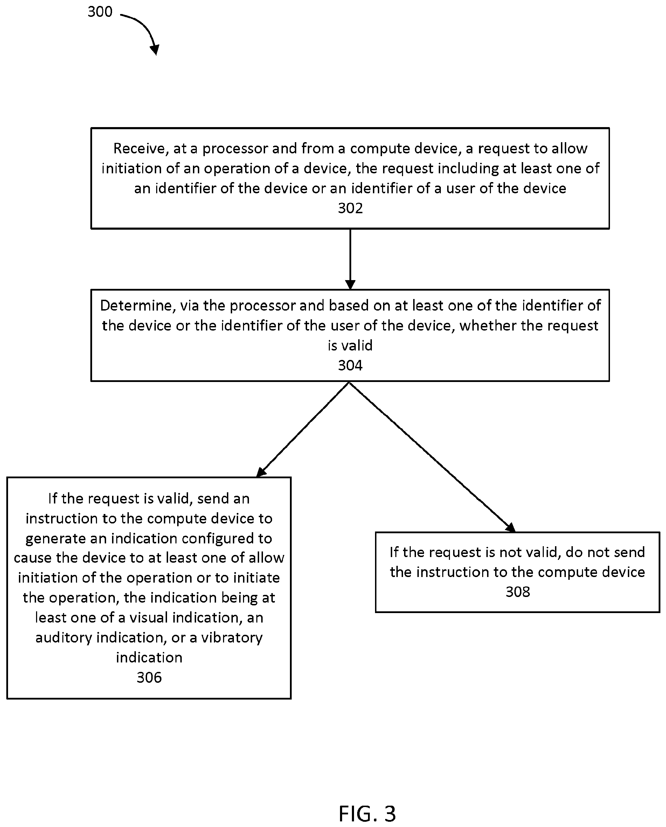

FIG. 3 illustrates a process-implemented method 300 of managing an operation of a device of a system, such as the device 101 of the system 100 described above. The method 300 includes receiving, at 302, at a processor and from a compute device, a request to allow initiation of an operation of a device. The request can include at least one of an identifier of the device or an identifier of a user of the device. At 304, the processor can determine whether the request is valid based on at least one of the identifier of the device or the identifier of the user of the device. At 306, if the request is valid, an instruction can be sent to the compute device to generate an indication configured to cause the device to at least one of allow initiation of the operation or to initiate the operation. The indication can be at least one of a visual indication, an auditory indication, or a vibratory indication. If the request is not valid, the instruction is not sent to the compute device.

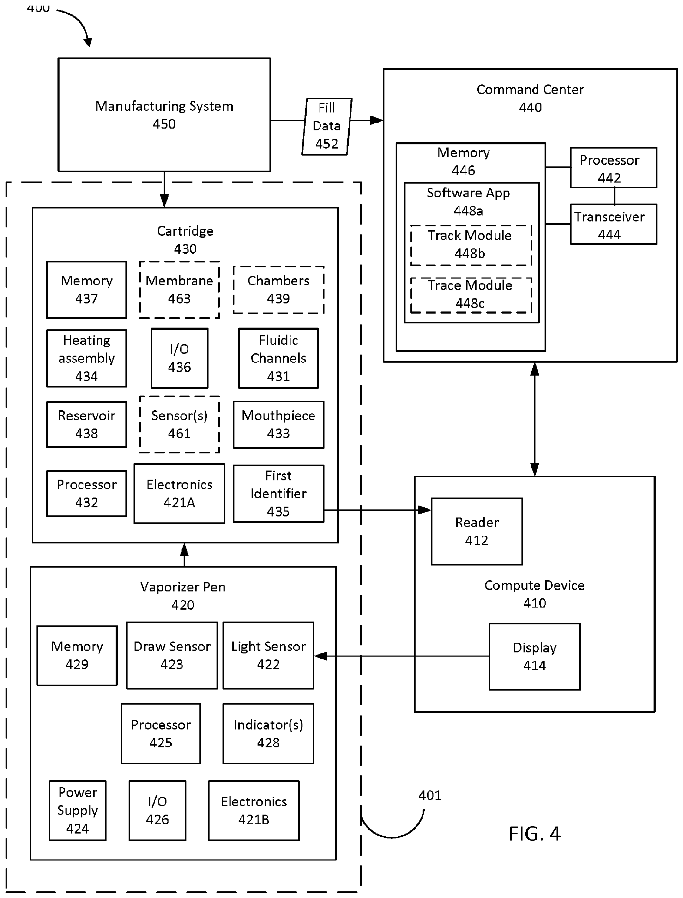

FIG. 4 is a schematic illustration of a system 400 for managing vaporizer cartridge activation, in accordance with some embodiments. The system 400 can be the same or similar in structure and/or function to any of the systems described herein, such as the system 100. As shown in FIG. 4, the system 400 includes a cartridge 430 and a vaporizer pen 420 that are configured to be coupled to form a vaporizer device 401. The vaporizer pen 420 and/or the vaporizer pen 420 and cartridge 430 in combination can be the same or similar in structure and/or function to the device 101 described above with respect to FIG. 1. The system 400 also includes a command center 440 (e.g., a cloud-based server, a centralized server and/or the like) in wireless network communication with a manufacturing system 450 (e.g., an empty cartridge manufacturing system, a filling system, and/or a packaging system) and a compute device 410 (e.g., a mobile device such as a smartphone or tablet, a laptop, or a desktop computer of the user). The command center 440 and the compute device 410 can be the same or similar in structure and/or function to any of the command centers and compute devices described herein, such as the command center 140 and the compute device 110.

The cartridge 430 (also referred to as a "cartridge assembly," a "cartridge portion," a "capsule," a "capsule assembly," or a "pod") includes a processor 432, a heating assembly 434, an input/output module 436 (referred to herein as "I/O"), a reservoir 438, fluidic channels 431, a mouthpiece 433, a first identifier 435, an optional memory 437, optionally one or more expansion chambers 439, electronics 421A, an optional membrane 463, and one or more optional sensors 461, all disposed within or coupled to a cartridge housing of the cartridge 430. The vaporizer pen 420 (also referred to as a "pen portion" or a "vaporizer body") includes a light sensor 422 (e.g., an RGB sensor), a draw sensor 423 (e.g., an airflow sensor or a pressure sensor), a power supply 424, a processor 425 (e.g., a microcontroller), an input/output module 426 (referred to herein as "I/O"), one or more indicators 428, and electronics 421B, all disposed within or coupled to a pen housing of the vaporizer pen 420. The vaporizer pen 420 can optionally include a memory 429. The vaporizer pen 420 can be reusable and includes an interface (e.g., including a portion of the electronics 421B) configured to electrically engage with the cartridge 430. The interface can include, for example, connectors (e.g., pogo pins) coupled to or included in the processor 425 (that may be coupled to the other electronics 421B) and configured to engage with the cartridge 430 such that the processor 432 of the cartridge 430 is configured to receive information from the processor 425 of the vaporizer pen 420 (e.g., based on, for example, data received via the light sensor 422) and operational power from the power supply 424 of the vaporizer pen 420. The vaporizer pen 420 (i.e., the pen housing and its contents) can also be referred to as a "battery portion."

The cartridge 430 can be manufactured, shipped and/or sold separately from the vaporizer pen 420, and assembled by a user to form the vaporizer device 401. To assemble the vaporizer device 401, a user may, prior to use (e.g., upon purchase of a new cartridge 430), couple (e.g., connect) the cartridge 430 with the vaporizer pen 420. The cartridge 430 and the vaporizer pen 420 can be configured to be mechanically connected, for example by one or more of screw attachment, press-fit attachment, snap-fit attachment, magnetic attachment, or any other suitable connection means. As can be inferred from the foregoing, the vaporizer pen 420 can be considered the reusable portion of the vaporizer device 401, and the cartridge 430 can be considered a disposable or "replaceable" portion of the vaporizer device 401. When the vaporizer pen 420 is coupled to the cartridge 430, under control of the processor 432 of the cartridge 430, the cartridge 430 can draw operational power from the power supply 424 of the vaporizer pen 420 (e.g., to power the heating assembly 434) via the interface.

The mouthpiece 433 of the cartridge 430 can comprise one or more of: ceramic, heat-resistant plastic, anodized aluminum, or any other suitable material. The reservoir 438 is configured to receive and contain carrier material (also referred to as "carrier" or "precursor"). The carrier material can include any suitable vaporizable substance. The reservoir 438 (also referred to as a precursor reservoir) can be in fluid communication with at least one of the mouthpiece 433, the one or more chambers 439 (e.g., vapor expansion chambers), and the fluidic channels 431, to facilitate the triggering of carrier heating and drawing of vapor in response to a user's creating a suction force (e.g., drawing) on the mouthpiece during use, for example using the draw sensor 461. For example, processor 432 of the cartridge 430 can be configured to activate the heating assembly 434 to heat the carrier in response to the processor 432 receiving a signal from the draw sensor 461 (e.g., via the processor 425 of the vaporizer pen 420) indicating that the draw sensor 461 sensed a change of pressure within the device 401 beyond a threshold change in pressure or a drop in pressure within the device 401 below a threshold pressure. Thus, when a user draws on an opening of the mouthpiece 433 causing a change in pressure within the device 401 that is sensed by the draw sensor 461, the processor 432 can activate the heating assembly 434.

The membrane(s) 463 can be disposed on an outer surface of the cartridge 430 (e.g., within an opening defined by a housing of the cartridge 430) and arranged such that carrier material and/or additive can be supplied to the reservoir 438 via the membrane(s) 463 (e.g., via insertion of a needle through the membrane). The membrane(s) 463 can include a valved impermeable or semi-permeable material, for example comprising a rubber, polyvinyl chloride (PVC), etc. The membrane(s) 463 can be resealable (e.g., automatically after removal of a needle). In some embodiments, rather than including a pierceable membrane 463, the outer surface of the cartridge 430 can define an opening via which carrier material and/or additive can be supplied to the reservoir 438. The opening can be selectively sealed via a plug (e.g., an elastomeric plug).

The heating assembly 434 includes a heating element and heater control circuitry configured to control the heating element. The heating element can include a coil heater, rod-shaped heater, pancake heater, chemical heater, a ceramic heater, and/or any other heater that is sized, dimensioned, and constituted of material suitable for heating the carrier material. In some implementations, the optional electronics 421A and/or the electronics 421B can include one or more of: a GPS receiver, one or more antennae, or a transmitter or transceiver for wireless (e.g., Bluetooth or WAN) communication with the command center 440 and/or other remote compute device (such as the compute device 410) (e.g., as an alternative method of communicating with the command center 440 regarding identifiers or keys and activation as described herein and/or to communicate various statuses of the cartridge 430 such as oil level, draw frequency, draw duration, etc.). The sensor(s) 461 can include one or more of: a pressure sensor, a temperature sensor, a position sensor, an orientation sensor, etc.

The first identifier 435 can be a visual identifier and can include any suitable data configured to identify the cartridge 430. The first identifier 435 can be represented via any suitable visual image, such as, for example, a serial number, a barcode, and/or a QR code such that the cartridge 430 may be identified and/or recognized by an external device (e.g., a fill station of the manufacturing system 450 and/or the compute device 410). In some implementations, the first identifier 435, displayed as a visual image, can be disposed on an outer surface of the cartridge 430 (e.g., a front surface, a side surface, or a back surface). For example, the first identifier 435 can be displayed on a label affixed to an outer surface of the cartridge 430. In some implementations, the first identifier 435 can be disposed on or included in a package containing the cartridge 430. In some implementations, the first identifier 435 can be scanned or read one or more of: during (or upon completion of) manufacturing, during (or upon completion of) filling, or when in possession of a user (e.g., scanned by a mobile device of the user, for example using a camera thereof, via a software application stored thereon). The first identifier 435 can thus be used for one or more of registration, identification, or validation/activation of the cartridge 430 (and/or a component thereof). In some implementations, the first identifier 435 can be unique to the particular cartridge 430 with which it is associated (i.e., each cartridge 430 configured to couple to the vaporizer pen 420 can have a distinct first identifier 435). In some implementations, the first identifier 435 can be unique to a particular subset of cartridges (e.g., cartridges sharing a common characteristic such as fill contents or fill date). In some implementations, the first identifier 435 can be generated as a randomized code. In some implementations, the first identifier 435 can include or be associated with a backup code. For example, the label on the cartridge 430 can include a first identifier 435 including both a QR code and a serial number. If the compute device 410 does not scan the QR code, or if the user prefers to input the serial number manually rather than scan the QR code, the user can input the serial number manually on a particular webpage to proceed with information gathering regarding the cartridge 430 and/or with the activation process. In some embodiments, rather than being disposed on the cartridge as a visual or visible identifier, the first identifier 435 can be digitally accessible. For example, in some embodiments, the first identifier 435 can be stored on a near-field communication (NFC) chip and can be transmitted to NFC-equipped devices through an NFC transmission. In some embodiments, the first identifier 435 can be stored in a memory of the cartridge 430 (e.g., the memory 437 or in a separate memory on a chip) and the cartridge 430 can be Bluetooth low energy (BLE) and/or WAN (e.g., LTE) enabled for transmission of the first identifier 435 to the compute device 410.

The processor 432 of the cartridge 430 can include one or more of: a general purpose processor, a central processing unit (CPU), a microprocessor, a digital signal processor (DSP), a controller, a microcontroller, a state machine and so forth. The processor 425 of the vaporizer pen 420 can include one or more of: a general purpose processor, a central processing unit (CPU), a microprocessor, a digital signal processor (DSP), a controller, a microcontroller, a state machine and so forth. Under some circumstances, a "processor" may refer to an application specific integrated circuit (ASIC), a programmable logic device (PLD), a field programmable gate array (FPGA), etc. The term "processor" may refer to a combination of processing devices, e.g., a combination of a DSP and a microprocessor, a plurality of microprocessors, one or more microprocessors in conjunction with a DSP core or any other such configuration. The processor 432 can be in electronic communication with the memory 437 and can be configured to read information from and/or write information to the memory 437. In some embodiments, the processor 432 and the memory 437 can be included on a common tracking component of the cartridge 430. The tracking component may be, for example, an integrated circuit (e.g., Application-Specific Integrated Circuits (ASICs)). Similarly, in some embodiments, the processor 425 can be in electronic communication with the memory 429 and can be configured to read information from and/or write information to the memory 429. The processor 425 and the memory 429 can be included on a common tracking component of the pen 420.

In some embodiments, the processor 432 can be configured to be loaded with a firmware during a manufacturing phase of the processor 432 or of the cartridge 430 such that the firmware can be programmatically used to perform authentication of the cartridge 432 using one or more cryptographic methods. The processor 432 can include a cartridge key (also referred to as a second identifier) stored in the memory 437 that is associated with the first identifier 435 and can be used during an authentication and/or validation process to unlock the cartridge 430 for operation. For example, in some implementations, the cartridge key can include a code or password that can be stored in the memory 437. The firmware of the processor 432 can include an authentication module and can be configured to access the stored code to verify that a key indicator of (e.g., included in or indicated by) a compute device presentation (also referred to as a third identifier) (e.g., a color, a sequence of colors, and/or a flashing sequence of color(s)) read by the light sensor 422 from the display 414 of the compute device 410 and provided to the processor 432 corresponds to the cartridge key. For example, the processor 432 can compare the cartridge key stored in the memory 437 of the tracking component to the key indicator of the compute device presentation presented by the display 414 and received by the processor 432 via the vaporizer pen 420 and can determine whether or not to activate the cartridge 430 based on whether the key indicator of the compute device presentation is based on (e.g., matches via a direct comparison, is a transformation of, is a derivative of, or corresponds to a one-way hash function of) the cartridge key. If the processor 432 determines that the key indicator of the compute device presentation is based on the cartridge key, the processor 432 can activate the cartridge 430 such that the processor 432 can activate the heating assembly 434 in response to a user drawing on the mouthpiece 433 (e.g., using operational powered provided from the power supply 424 of the vaporizer pen 420 to the processor 432). If the processor 432 does not determine that the key indicator of the compute device presentation is based on the cartridge key, the cartridge 430 will not be activated such that the processor 432 is not able to activate the heating assembly 434 in response to a user drawing on the mouthpiece (even if operational power is available to due to the processor 432 being electrically coupled to the power supply 424 of the vaporizer pen 420). In some implementations, the cartridge key can include a digital signature (also referred to as an authentication signature) stored in the memory 437 (e.g., of the tracking component such as a chip) of the cartridge 430 that can be based on a private key, and the processor 432 can determine whether to activate the cartridge 430 such that the processor 432 can activate the heating assembly 434 in response to a user drawing on the cartridge 430 based on a similar method as described with respect to authenticating and validating a tracking component in U.S. Patent Publication No. 2020/0085105 to Mladen Barbaric et al., filed on September 48, 2019 and entitled Methods and Systems for Vaporizer Security and Traceability Management and/or U.S. Patent Publication No. 2020/0113246 to Mladen Barbaric et al., filed on October 46, 2019 and entitled Variable-Viscosity Carrier Vaporizers with Enhanced Thermal and Hydrodynamic Properties. Although the light sensor 422 is described as a light sensor and the compute device presentation is described as being a visual compute device presentation, in some embodiments, the sensor 422 can include any of the sensors described herein (such as, for example, with respect to the sensor 122) and the compute device presentation can be in any form able to be sensed by the sensors as described herein (such as, with respect to the system 100).

In some embodiments, rather than the cartridge 430 storing the cartridge key on the memory 437 and the processor 432 determining if a sensed key indicator is based on the cartridge key as described above, the vaporizer pen 429 can have a key (e.g., a device key or a vaporizer key) stored on the memory 429 of the vaporizer pen 420. The processor 425 can function the same or similarly as the processor 432 described above. For example, the processor 425 of the vaporizer pen 420 can determine whether to activate the cartridge 430, to activate the pen 420, and/or to provide power from the power supply 424 to the heating assembly 434 based on whether the key stored on the memory 429 is based on the key indicator in an indication (e.g., of a compute device presentation) sensed by the sensor 422. Additionally, the processor 425 can make the determination whether to activate, validate, initiate an operation or, or allow initiation of an operation of the cartridge 430 and/or the pen 420 based on any of the conditions described herein with respect to other processors, such as those described with respect to the system 100 and/or above with respect to the system 400 (e.g., based on user identifiers including identity, age, prescription, membership, and/or subscription checks, based on a recall status of the cartridge 430 or pen 420, based on a geographic location of the cartridge 430, pen 420, or compute device 410, based on a time since last validation or likeness check, and/or any other conditions described herein). In some embodiments, the key (e.g., a cartridge key) can be stored on the memory 437 of the cartridge 430 and retrieved by the processor 425 of the pen 420 when the cartridge 430 is electrically engaged with the pen 420, such that the processor can function similarly to the processor 432 described above. In some embodiments, the heating assembly 434 configured to heat the contents of the reservoir 438 can be partially or completely included in (e.g., disposed within) the vaporizer pen 420 rather than within the cartridge 430. For example, in some embodiments, the reservoir 438 can be configured to contain dry herbs or a solid substance for vaporization, and the cartridge 430 can be coupled to the pen 420 including the heating assembly 434 for heating of the reservoir 438 of the cartridge 430 under control of the processor 425. In some embodiments, the cartridge 430 does not include the processor 432, the memory 437, and/or the electronics 421A, and may optionally include the first identifier 435 for scanning or other identification by the compute device 410. In some embodiments, the cartridge 430 may include only the reservoir 438, a housing, and optional fluidic channels. In some embodiments, the cartridge 430 may include only the reservoir 438, a housing, the mouthpiece 433, and optional fluidic channels. In some embodiments, the cartridge 430 may include only the reservoir 438, a housing, a mouthpiece, optional fluidic channels, and a first identifier 435. In some embodiments, the device 401 can be a unitary device such that the user does not engage or disengage the cartridge 430 from the pen 420 prior to or during usage. In such cases, the device 401 may only have one of the memories 437, 429 and one of the processors 425, 432. In some embodiments, the device 401 is formed as a unitary and disposable (e.g., after delivery or dispensing of the contents of the reservoir 438) and does not include distinct pen and cartridge portions separable and engageable by a user. For example, the components of the device 401 can be all included in a single outer housing. In some embodiments, the outer housing and a mouthpiece of the vaporizer device can be formed such that a user cannot separate them without breaking the device (e.g., formed monolithically). In embodiments in which the device 401 is formed as a unitary vaporizer device, the device 401 can be activated, validated, and/or operated the same or similarly to any of the devices described herein (e.g., can include a device key stored on the memory 429 and can have a processor 425 configured to determine if a key indicator sensed by the sensor 422 is based on the device key). Additionally, the device 401 can include a device identifier that can be the same or similar to any of the device identifiers described herein (e.g., device identifier 435) that can be read by the compute device 410 and provided to the command center 440 (e.g., with a request relating to usage of the device 401) for the command center 440 to use to determine whether to instruct the compute device 410 to present an indication (e.g., within a compute device presentation) associated with approving the request (e.g., associated with validating, activating, and/or operating the device 401).

In some embodiments, the cartridge 430 and/or the vaporizer pen 420 can include a challenge-response mechanism such that the cartridge 430 (e.g., the processor 432) will only determine whether to activate after receiving a return communication from the vaporizer pen 420 (e.g., from the processor 425 of the vaporizer pen 420) including or based on a challenge previously provided to the vaporizer pen 420 from the cartridge 430. If the vaporizer pen 420 (or another vaporizer pen) attempts to send the key indicator or a message based on the key indicator to the cartridge 430 without the challenge value, the cartridge 430 will not accept any activation attempt from the vaporizer pen 420, even if the activation attempt includes or is based on a key indicator that is based on the cartridge key. For example, in response to the light sensor 422 reading the compute device presentation presented on the display 414 of the compute device 410, the vaporizer pen 420 can transmit a request for a challenge (e.g., a value) from the cartridge 430. In response to receiving the request for a challenge from the vaporizer pen 420, the cartridge 430 can transmit the challenge to the vaporizer pen 420. In response to receiving the challenge, the vaporizer pen 420 can transmit a response including a combination (e.g., a hash) based on the challenge and the key indicator of the compute device presentation to the cartridge 430. The cartridge 430 can then determine whether the response includes the challenge that was sent from the cartridge 430 to the vaporizer pen 420. If so, the cartridge 430 can determine whether the key indicator is based on the cartridge key (e.g., whether the cartridge key corresponds to the key indicator or whether a hash based on the key indicator corresponds to the key indicator). If so, the cartridge 430 can determine that the hash is valid and activate such that the processor 432 can activate the heating assembly 434 in response to a user drawing on the mouthpiece 433. Such a challenge-response mechanism and process can be included and/or implemented by any of the devices described herein, such as and of the two portion devices described with respect to the device 101 of FIG. 1.

In some embodiments, the compute device presentation can include a calibration sequence presented prior to the color, sequence of colors, and/or flashing sequence of color(s) associated with the key indicator. The calibration sequence can be used to ensure that the light read by the light sensor 422 of the vaporizer pen 420 is accurately transmitted to the cartridge 430 regardless of the variation or distortion in color or tint of a display 414 of the compute device 410 compared to other compute devices. The calibration sequence can include a predetermined sequence of colors (e.g., including each color used in the portion of the compute device presentation associated with the key indicator). The vaporizer pen 420 (e.g., the processor 425 of the vaporizer pen 420) can be configured to recognize the colors in the calibration sequence based on the predetermined sequence. The vaporizer pen 420 (e.g., the processor 425 of the vaporizer pen 420) can be configured to identify the key indicator based on the colors in the portion of the compute device presentation associated with the key indicator based on the colors in the calibration sequence. In some embodiments, the compute device presentation can include a delimiter at the beginning and end of the sequence (e.g., prior to the calibration sequence and after the portion of the compute device presentation associated with the key indicator) such that the vaporizer device 420 can identify the beginning and end of the compute device presentation. In some embodiments, the compute device presentation can include any suitable number of colors (e.g., two, three, four, five, six, seven, eight, nine). For example, the compute device presentation can include six non-black colors and black.

The memory 437 and/or the memory 429 can include any electronic component capable of storing electronic information. The term memory may refer to various types of processor-readable media such as random access memory (RAM), read-only memory (ROM), non-volatile random access memory (NVRAM), programmable read-only memory (PROM), erasable programmable read only memory (EPROM), electrically erasable PROM (EEPROM), flash memory, magnetic or optical data storage, registers, etc. Memory is said to be in electronic communication with a processor if the processor can read information from and/or write information to the memory. Memory that is integral to a processor is in electronic communication with the processor. Although not shown in FIG. 4, each of the vaporizer pen 420 and/or the cartridge 430 can also include any additional memory needed such that the vaporizer pen 420 and/or the cartridge 430 is operational to perform the functions of the vaporizer pen 420 and/or cartridge 430 described herein.

The power supply 424 of the vaporizer pen 420 can include any suitable battery or fuel cell, for example having high-drain characteristics. In some implementations, the vaporizer pen 420 can include a mechanical interface (e.g., a button) as part of the I/O 426 that the user can actuate to trigger the heating and vaporization of the carrier. The input/output module 426 can include one or more of: a push-button control for causing vapor generation (as an alternative to activating the heating assembly 434 based on the draw sensor 423), a battery indicator, an electromechanical connector for charging and/or data communication, a light source (e.g., one or more light-emitting diodes), etc. The indicator(s) 428 can include one or more of: an illumination source (e.g., one or more light-emitting diodes), a speaker, a display screen, a vibration component (e.g., a vibration motor or a piezoelectric vibrating element), etc. In some embodiments, one or more of the indicator(s) 428 can be included in or controlled by a component of the input/output module 426.

The manufacturing system 450 can be the same or similar in structure and/or function to any of the manufacturing systems described herein, such as the manufacturing system 150. The manufacturing system 450 can include a memory operably coupled to a processor. The memory can store data (e.g., in the form of a database table storing data records) associated with one or more of: carrier formulations, carrier provenance, capsule compatibility, capsules that have been filled at a filler station of the manufacturing system 450 (e.g., capsule identifier(s)), filler station maintenance history, filler station maintenance schedules, and fill settings such as carrier dispense pressure, carrier dispense temperature, carrier dispense duration, carrier dispense volume, carrier dispense regimens (e.g., milligrams per day) etc. The manufacturing system 450 also includes a transceiver operably coupled to the processor and the memory. The transceiver facilitates communications between the manufacturing system 450 and the command center 440. For example, the manufacturing system 450 (e.g., a filler station of the manufacturing system 450) may send fill data 452 to the command center 440. Example fill data 452 include, but are not limited to, identifiers of one or more of: capsule type, capsule capacity, carrier type, carrier amount, carrier origin/provenance, carrier constituent concentration(s), fill date/time stamp, fill conditions, etc. While not shown in FIG. 4, the manufacturing system 450 can include one or more manufacturing stations or manufacturing jigs that are configured to manufacture empty/unfilled capsules. In some embodiments, the manufacturing station used to manufacture a capsule can be separately located and operated from a filling station of the manufacturing system 450 used to fill the capsule (e.g., located in a different facility under the control of a different organization). In some embodiments, the manufacturing station and the filling station may be co-located, combined, and/or under the control of the same organization. The manufacturing station can be configured to store a cartridge key (e.g., write a code or write a digital signature using a private key based on the cartridge key) in the memory 437 included in each cartridge 430 and/or place the first identifier 435 (e.g., a QR code, bar code) on an external surface of each capsule for registration, verification and/or validation at various steps downstream in the process of making and using the capsule. The fill data 452, the first identifier 435, and the cartridge key can be associated with one another in the command center 440. For example, a manufacturing station of an empty cartridge 430 can send the first identifier 435 and the cartridge key to the command center 440 such that they are associated in a database in a memory 446 of the command center 440. A filler station (e.g., related to or distinct from the manufacturing station of the manufacturing system 450) can read (e.g., scan) the first identifier 435 and associate first identifier 435 with the fill data 452 associated with the cartridge 430 before, during, or after filling the cartridge 430 when communicating the fill data 452 to the command center 440 (e.g., by including the first identifier 435 with the fill data 452). Thus, the command center 440 can include a database in a memory 446 of the command center 440 associating the first identifier, the cartridge key, and the fill data 452 associated with the cartridge 430. In some embodiments, the cartridge key is unique to only one cartridge 430. In some embodiments, the cartridge key can be stored in the memory of more than one cartridge 430. In some embodiments, the cartridge key can be stored in the memory of more than one cartridge 430, but used in only a small portion of manufactured cartridges 430 (e.g., in only one or in only a small number of a manufactured lot) (e.g., less than 5%, less than 4%). As noted above, in some embodiments, the vaporizer pen 420 can be associated with a vaporizer key stored on the memory 429 that can be the same or similar to the cartridge key described above (e.g., with respect to relationships with the command center 440 and/or the manufacturing system 450).

The command center 440 includes a memory 446 (as referenced above) operably coupled to a processor 442, and a transceiver 444 configured to facilitate wireless network communications with the manufacturing system 450 and the compute device 410 of the user. For example, messages related to user registration and/or vapor cartridge validation/activation (e.g., a cartridge activation request message) can be exchanged between the command center 440 and the compute device 410.

As discussed above, in some embodiments, the cartridge 430 will not operate when coupled to the vaporizer pen 420 unless the cartridge 430 has first been validated and activated. In use, a user can use the compute device 410 to read the first identifier 435 disposed on the outer surface of the cartridge 430. For example, the compute device 410 (e.g., a smartphone) can include a reader 412 (e.g., a camera and related software application stored on the compute device 410) to read the first identifier 435 (e.g., a QR code). In some embodiments, in response to reading the first identifier 435, the compute device 410 can communicate with the command center 440 to retrieve information associated with the cartridge 430 (e.g., based on or including the fill data 452) by sending data including the first identifier 435 to the command center 440. In response to receiving the information associated with the cartridge 430, the compute device 410 can present an initial informative presentation (e.g., a webpage or an application) on a display 414 of the compute device 410 (e.g., automatically) including at least some of the information associated with the cartridge 430. Thus, the user can view information associated with the cartridge 430, such as a source of the contents of the reservoir 438, concentration, dosage, and/or ingredients of the contents of the reservoir 438, flavor data of the contents of the reservoir 438, etc. In some embodiments, rather than reading the first identifier 435 with the reader 412 of the compute device 410, the user can manually input a serial code associated with the first identifier 435 (e.g., printed near the QR code) and the compute device 410 can retrieve information associated with the cartridge 430 based on the serial code.

In some embodiments, the initial informative presentation on the display 414 can include an interaction feature (e.g., a touch button on the screen) configured for user interaction. If, after reviewing the initial informative presentation, the user desires to activate the cartridge 430, the user can select the interaction feature (e.g., press on the button). In response to the user selecting the interaction feature, the compute device 410 can send a cartridge activation request message to the command center 440. The command center 440 can respond by sending instructions to the compute device to present the compute device presentation on the display 414 of the compute device 410. The compute device presentation can include a key indicator based on the cartridge key that is associated with the first identifier 435 in the memory 446 of the command center 440. The compute device presentation can include a particular color or series of colors displayed over a portion or all of the display 414. The key indicator of the compute device presentation can be based on a conversion of the cartridge key stored as a binary code in the command center 440 to a base-6 code, for example, that can be communicated through a series of colors and/or series of flashes of one or more colors. In some embodiments, rather than the command center 440 first sending instructions to the compute device 410 to present a presentation on the display 414 including information associated with the cartridge 430 (e.g., the initial informative presentation) and/or sending instructions to display the compute device presentation in response to the user selecting the interaction feature, the compute device 410 can send the cartridge activation request message to the command center 440 in response to initially scanning the first identifier 435 and the command center 440 can send the instructions to present the compute device presentation including the key indicator to the compute device 410 with and/or instead of the instructions to present information associated with the cartridge 430 (e.g., the initial informative presentation).

After scanning the identifier 435 with the reader 412 of the compute device 110, the user can engage the cartridge 430 with the vaporizer pen 420 (e.g., insert the cartridge 430 into an interior space of the vaporizer pen 420 to engage with an interface of the vaporizer pen 420 to establish a mechanical and electrical connection between the cartridge 430 and the vaporizer pen 420). In some embodiments, the vaporizer pen 420 can indicate that the cartridge 430 has been coupled to the vaporizer pen 420 via the indicator(s) 428 (e.g., via flashing an LED such as with rainbow colors). The user can interact with the compute device 410 to request activation, validation, or another operation with respect to the cartridge 430 using a webpage or application of the compute device 410 (which may automatically be displayed on the compute device 410 display screen in response to scanning the identifier 435 with the reader 412). Optionally, the webpage or application can then instruct the user to place the vaporizer pen 420 directly in contact with the display screen of the compute device 410, and may indicate a target contact location or area. With the cartridge 430 engaged with the vaporizer pen 420, the user can arrange the vaporizer pen 420 against the display 414 of the compute device 410 such that the light sensor 422 can read the display 414 of the compute device 410. For example, the side of the vaporizer pen 422 on which the light sensor 422 is disposed can be placed adjacent a screen of the display 414 such that the light sensor 422 is aligned with the compute device presentation to read the color(s) and/or series of color(s) flashed and/or displayed on the display 414. The indicator(s) 428 may be used as orientation indicators. For example, the indicator(s) 428 can be disposed on a side of the vaporizer pen 420 opposite a side including or coupled to the light sensor 422. The user can confirm that the vaporizer pen 420 has been properly placed on the display screen by confirming that the indicator(s) 428 are on a side of the vaporizer pen 420 facing away from the display screen and/or facing upward when the compute device 410 is disposed on a table or other surface with the display screen facing up and the vaporizer pen 420 is properly disposed directly on the display screen. In some embodiments, the initial informative presentation includes instructions for the user as to how to arrange the vaporizer pen 420 relative to the display 414 of the compute device 410 prior to the presentation of the compute device presentation. In some embodiments, after disposing the vaporizer pen 420 in contact with the display screen of the compute device 110, the user can interact with the display screen (e.g., press a button) to instruct the display screen to proceed with presenting the compute device presentation.

Upon the light sensor 422 reading the compute device presentation, the processor 425 of the vaporizer pen 420 can communicate some or all of the content of the compute device presentation to the processor 432 of the cartridge 430. In some embodiments, prior to communicating the content of the compute device presentation (e.g., the key indicator or a message based on the key indicator) to the processor 425 of the vaporizer pen 420, the processor 425 can request a challenge from the processor 432 of the cartridge 430 to be included with the content or to be transmitted prior to communicating the content, as described above. The processor 432 of the cartridge 430 can determine (e.g., using operational power from the power supply 424) if the key indicator (e.g., code) provided via the compute device presentation is based on the cartridge key (e.g., matches the cartridge key or is a derivative of the cartridge key) saved on the memory 437. If the key indicator is confirmed to match the cartridge key, the processor 432 can transition the cartridge 430 from a locked status to an unlocked status (e.g., activate the cartridge 430). Upon unlocking (e.g., activation), the cartridge 430 can be used by the user for vaporization of the contents of the reservoir 438. For example, the processor 432 can initiate the heating assembly 434 (e.g., in response to a signal from the draw sensor) using operational power from the power supply 424 of the vaporizer pen 420 to power the heating assembly 434. In some embodiments, after unlocking, the cartridge 430 can be used with other vaporizer pens 420 than the vaporizer pen 420 used to unlock the cartridge 430. In some embodiments, the compute device presentation can also include fill information, heating instructions, and/or other information associated with the contents of the reservoir 438, such as if the compute device presentation is the compute device presentation associated with the first activation of the device 401 or a component thereof. Thus, the device 401 or the component thereof can be synchronized with the most up to date operation instructions. For example, the compute device presentation can include a heating profile and a rate of release of the carrier material and/or one or more ingredients of the carrier material (e.g., based on the heating profile) associated with the cartridge 430 in addition to including the code associated with the key indicator such that the processor 425 of the vaporizer pen 420 can write the heating profile and rate of release on the memory 437 of the cartridge 430 or can send instructions to the processor 432 to write the heating profile and rate of release on the memory 437 of the cartridge 430. The heating profile can be specific to the carrier material in the reservoir 438. Thus, upon activation of the cartridge 430, the heating assembly 434 can heat the contents of the reservoir 438 to one or more target temperatures at one or more rates for one or more durations according to the heating profile and can provide indications to the user via the indicators 428 (e.g., LED-based and/or haptic) representing characteristics of the cartridge 430 (e.g., fill level of the reservoir 438) and/or characteristics of usage of the cartridge 430 (e.g., draw strength of a draw on the mouthpiece 433 based on, for example, an amount of an ingredient of the vaporized carrier material drawn by the user). In some embodiments, the compute device presentation can also include a time-based or usage-based (e.g., draw-based) limit or lock out instruction as described above with respect to the system 100 such that the pen 420 and/or the cartridge 430 will stop operating when the limit is reached until the cartridge 430 (or the pen 420) has been re-validated (e.g., via completing the requirements of a likeness check as described above with respect to the system 100). In some embodiments, the cartridge 430 and/ or the pen 420 can be counting the usage or time with respect to the limit (e.g., the processor 432 and/or the processor 425 can include counters or timers) so that the cartridge 430 and/or the pen 420 cannot be used with another pen or cartridge, respectively, after the lock out has initiated.

In some embodiments, the command center 440 can provide instructions to display the compute device presentation to the compute device 410 only if the user has an active subscription or membership status (i.e., only if the subscription status of the user has been validated). For example, upon the user reading the first identifier 435 with the reader 412 of the compute device 410, the compute device 410 can send the first identifier 435 to the command center 440 with a user identifier, a vaporizer device identifier, and/or a mobile device identifier associated with the user, the vaporizer device, and the mobile device, respectively. The user identifier, the vaporizer device identifier, and/or the mobile device identifier may be based on, for example, a log-in or account number assigned to the user or the mobile device usable to identify an account associated with the user and/or the mobile device. The command center 440 can determine if the user identifier, the vaporizer device identifier, and/or the mobile device identifier are associated with an active subscription. If the user identifier, the vaporizer device identifier, and/or the mobile device identifier is associated with an active subscription, the command center 440 can proceed with sending the instructions to display the compute device presentation on the display 414. If the user identifier, the vaporizer device identifier, and/or the mobile device identifier are not associated with an active subscription (e.g., a previous subscription has lapsed or a new subscription needs to be established), the command center can send a communication to the compute device 410 including instructions to display a prompt for the user to subscribe or resubscribe (e.g., submit funds and/or create or update an account having a subscription). Once the user has taken the steps required to change the subscription status stored in the command center 440 from inactive to active (e.g., via engagement with the compute device 410), the compute device 410 can communicate the instructions to display the compute device presentation on the display 414 of the compute device 410 to the compute device 410.

In some embodiments, the command center 440 can provide instructions to display the compute device presentation including the key indicator to the compute device 410 only if the compute device identifier corresponds to an approved, validated, or registered compute device identifier in the memory 446 of the command center 440. If the command center 440 determines that the compute device identifier is not registered, the command center 440 can transmit an instruction to the mobile device 410 to display instructions for registration of the compute device 410. In some embodiments, the command center 440 can provide instructions to display the compute device presentation including the key indicator to the compute device 410 only if the vaporizer pen identifier corresponds to an approved, validated, or registered vaporizer pen identifier in the memory 446 of the command center 440. If the command center 440 determines that the vaporizer pen identifier is not registered, the command center 440 can transmit an instruction to the mobile device 410 to display instructions for registration of the vaporizer pen 420. In some embodiments, the command center 440 can provide instructions to display the compute device presentation including the key indicator to the compute device 410 only if the user identifier is associated with the cartridge key, key indicator, and/or vaporizer pen identifier stored in the memory 446 of the command center 440. In some embodiments, the command center 440 can provide instructions to display the compute device presentation including the key indicator to the compute device 410 only if the cartridge activation request is sent from the compute device 410 to the command center 440 within a particular window of time (e.g., before a cutoff date or time and/or after a cutoff date or time). Thus, one or more cartridges 430 can be intended for particular predetermined time periods (e.g., by a medical practitioner) and will not be able to be activated outside of the predetermined time periods. In some embodiments, a medical practitioner can transmit a prescription record or prescription update to the command center 440 to associate a prescription status and/or prescription restrictions (e.g., dosage amount and schedule) with a user identifier, a compute device identifier, a first identifier, and/or a vaporizer pen identifier. In some embodiments, only a predetermined number of cartridges may be activated during the predetermined time period. In some embodiments, the command center 440 can implement a time-based restriction between activations of cartridges 430 or cartridges 430 having the same type of carrier material such that the activations are spaced apart to prevent over usage. In some embodiments, the command center 440 can provide instructions to display the compute device presentation including the key indicator to the compute device 410 only if the provenance record associated with the first identifier 435 can be located and verified to match the first identifier 435.

In some embodiments, a user and/or medical practitioner can set a weaning or quit plan (e.g., using the compute device 410 or another compute device in communication with the command center 440). The weaning or quit plan can set a limit on draw volume and/or dosage for a particular time period (e.g., a portion of a day, a day, a week, a month) and can repeat or vary over a set of time periods (e.g., different limits for different days of the week and/or decreasing limits over time). The compute device 410 can include the time-based limits in a compute device presentation generated by the compute device 410 (e.g., under instruction from the command center 440) such that the processor 425 of the pen 420 or the processor 432 of the cartridge 430 can implement the limits. The processor 425 of the pen 420 or the processor 432 of the cartridge 430 can be configured to track usage (e.g., based on draw volume or count) and stop access (e.g., operation of the heating assembly 434) for a particular time period if the limit is reached.

In some embodiments, the processor 432 of the cartridge 430 can include a fraud mitigation feature to avoid a brute force activation in which a user may attempt to activate the cartridge 430 by aligning the light sensor 422 with a display displaying or flashing a color or series of colors (e.g., a randomized display). The fraud mitigation feature may include a lock-out feature such that, in the event of a certain number of failed activation attempts (e.g., one, two, three) in which the light sensor 422 was aligned with a display but the processor 432 did not identify a match between the cartridge key and the data or code transmitted from the display via the light sensor 422 (e.g., the key indicator), the processor 432 can pause or lock for a predetermined period of time (e.g., thirty seconds, two minutes, three minutes) before another activation attempt can be initiated. In some embodiments, the predetermined period of time can progressively increase with each failed activation attempt, such that the first pause of lock period is shorter than the duration of the second, which is shorter than the duration of the third, etc. For example, the first pause period can be thirty seconds, the second can be two minutes, the third can be ten minutes, the fourth can be an hour, the fifth can be six hours, etc. In some embodiments, in each instance that the processor 432 of the cartridge 430 does not activate as a result of an activation attempt by the vaporizer pen 420 (e.g., due to a mismatch between the cartridge key and the key indicator), an activation counter of the cartridge 430 can increase an increment of the activation counter by one. If the activation counter reaches a predetermined threshold value, the cartridge 430 will permanently disable itself to prevent a malicious party from attempting to gain unauthorized access to the cartridge 430 and its contents (e.g., carrier material).

The memory 446 of the command center 440 can store a software application ("app") 448a. In some implementations, an administrator of the command center 440 interacts with the software app 448a via an administrator view of the app, rendered via a graphical user interface (GUI) of a compute device in wireless or wired network communication therewith, and a user interacts with the software app 448a via a user view of the app, rendered via a graphical user interface (GUI) of a compute device (e.g., the display 414 of the compute device 410) of the user in wireless network communication with the command center 440. The app 448a can include one or more software modules, such as a track module 448b and/or a trace module 448c.

The track module 448b can include instructions to cause the processor 442 to obtain contemporaneous (e.g., real-time or substantially real-time) location information for one or more vaporizer components (e.g., cartridge 430) and/or one or more compute devices (e.g., the compute device 410) of a user. Such location can be obtained, for example, by querying one or more of the aforementioned devices (e.g., via one or more associated onboard location sensors of the device(s), such as a global positioning sensor (GPS) receiver). The track module 448b can also include instructions to cause the processor 442 to store the location information and, optionally, transmit the location information to one or more requestors of the wireless network (e.g., requestors associated with remote compute devices such as compute device 410 or a third party).

For example, some substances (e.g., controlled substances) that may be included in one or more carrier materials and consumable via the vaporizer pen 420 and cartridge 430 may be lawfully consumed in certain geographical locations whereas consumption of the substances may not legally be permitted in other geographical locations. When the reader 412 of the compute device 410 reads the first identifier 435 of the cartridge 430, the track module 448b can receive (e.g., via the compute device 410 based on a geographical location of the compute device 410) contemporaneous location information associated with the cartridge 430. In some implementations, the track module 448b can have access to information regarding location-based permissions and/or restrictions with respect to the consumption of specific substances. Based on the location-based restriction information, the location of the cartridge or the compute device 410, and information regarding the constituent substances included in the carrier material in the cartridge, the track module 448b can determine whether operation of the cartridge to volatilize the carrier material in the capsule will be permitted. The processor 442 can then send instructions to the compute device 410 via the transceiver 444 based on the determination. For example, the instructions can permit the use of the cartridge 430 by validation of the user and/or the cartridge 430 (e.g., by sending instructions to display the compute device presentation to the compute device 410), or can block or disable the use of the cartridge 430 by not validating the user and/or the cartridge 430. In some embodiments, based on the geographical location or any of the other conditions described herein, the pen 420 can be blocked or disabled (e.g., via not validating the pen 420 in embodiments in which pen validation is necessary for operation or via the sensor 422 sensing a compute device presentation generated by the compute device 110 including an instruction understandable by the processor 425 to cause the pen 420 to not operate or become disabled).

In some implementations, the track module 448b can receive updated information regarding the location of the cartridge 430 or the compute device 410 including the controlled substance, and based on the updated location information, the track module 448b can update instructions related to permissions of usage. For example, based on updated location information from the reader 412 of the compute device 410 (or a different compute device 410) in a different permissible location, the track module 448b can unblock the use of a blocked cartridge 430 by validating the user (e.g., via the compute device 410) and/or the cartridge 430 and sending instructions to display the compute device presentation on the display 414 of the compute device 410.

The trace module 448c can include instructions to cause the processor 442 to request, store and/or transmit historical data associated with the manufacture and movement (e.g., within the supply chain), of one or more vaporizer components (e.g., capsules or vaporizer pens, such as the cartridge 430) and/or one or more compute devices (e.g., the compute device 410) of the user. In other words, the trace module 448c (optionally in combination with the track module 448b) monitors the chain-of-custody of one or more cartridges 430 to ensure their safety and authenticity. The historical data can include one or more of: carrier ingredients, carrier formulation, nicotine concentration, nicotine plant genetics, nicotine provenance data (e.g., the tobacco plant(s) from which the nicotine was derived, the grow location of the nicotine plant(s), the grow and/or harvesting date of the nicotine plant(s), etc.) cannabinoid concentration(s), cannabinoid provenance data (e.g., the cannabis plant(s) from which the cannabinoid(s) were derived, the grow location of the cannabis plant(s), seed information associated with the cannabis plant(s), the date on which the cannabis seeds were planted, the grow and/or harvesting date of the cannabis plant(s), the dispensary from which the cannabinoid(s) were obtained, etc.), active ingredient (e.g., drug) concentration, extraction method(s) (and details thereof) used when converting the cannabis plant(s) into carrier material, inactive ingredient concentration, functionality of the vaporizer (e.g., physics of vapor generation, sequence of steps performed by the vaporizer when activated, etc.), details regarding effects within/on the user when the vapor is inhaled, and/or the like. In some implementations, the historical data (or a subset thereof) is rendered via a graphical user interface (GUI) (e.g., via the display 414 as described above with reference to the presentation) for presentation to a user, e.g., via a software application running on the compute device 410.

In some implementations, the trace module 448c can be configured to generate and maintain a list or database of cartridges (e.g., cartridge 430). In some implementations, the trace module 448c can (optionally in combination with the track module 448b) maintain a list or database of cartridges that were authenticated at or after filling (e.g., by a filler station) and a list of capsule identifiers associated with the authenticated capsules. In some implementations, as described above, the trace module 448c can maintain a list or database of first identifiers (e.g., visual identifiers or codes associated with and representable by visual identifiers) associated with cartridge keys (e.g., codes or electronic identifiers) and/or key indicators (e.g., color-based identifiers). Each cartridge 430 can be identified by the trace module 448c via the first identifier. In some implementations, the trace module 448c can maintain a list or database of cartridges 430 that can be authenticated and authorized for use (e.g., unlocked using a compute device 410) (e.g., by verifying the cartridge key stored on the processor 432 of the cartridge 430 corresponds to the cartridge key stored in the memory of the command center 440 and represented by the key indicator of the compute device presentation displayed via the display 414 of the compute device 410).

In some implementations, at any point in time a set of cartridges and/or carrier material disposed in the set of cartridges can be identified as being faulty or can undergo a regulatory restriction of use (e.g., restriction of use in a specific region or by a specific user group based on, for example, age). The trace module 448c can be used to generate a recall list or a block list including cartridge identifiers associated with each of the cartridges in the set. In some embodiments, the trace module 448c can associate a recall identifier with a specific cartridge identifier (e.g., a first identifier) or carrier identifier. When an incoming request for verification or validation of a capsule is received by the command center 440 (e.g., a cartridge information or activation request including the first identifier 435 of the capsule 430 from the compute device 410), the trace module 448c can be configured to determine whether the first identifier 435 of the cartridge 430 is on the recall list or has been associated with a recall flag or indication. If the first identifier 435 is determined to be on the recall list, the trace module 448c can block the validation of the cartridge 430 associated with the recall. Thus, in some embodiments, in case of a recall associated with one batch of cartridges for example, the system described herein can be used to block a cartridge 430 from being validated at a first instance of the first identifier 435 of the cartridge 430 being transmitted from the compute device 410 to the command center 440 (e.g., after being read off the cartridge 430 by the reader 412) such that the instructions to display the compute device presentation including the key indicator will not be provided to the compute device 410. In some embodiments, the command center 440 may provide instructions via the transceiver 444 to the compute device 410 to display information on the display 414 associated with the recall and/or additional instructions.

In some instances, the trace module 448c can receive a request for user verification and based on a determination of a user characteristic (e.g., an age of a user), the trace module 448c can block the verification of the user for a specific capsule associated with the first identifier 435 (e.g., by not sending instructions to display the compute device presentation including the key indicator to the compute device 410). In some implementations, the user validation or verification can include uploading an image of a government-issued identification card for review by the control center 440. The control center 440 can determine the age of the user based on the image. In some implementations, the trace module 448c can be configured to implement a substance block for a particular user based on the user verification such that particular substances (e.g., associated with particular carrier identifiers) can be validated for use by the user and other substances cannot be validated (e.g., based on regulatory age restrictions). Upon validating or verifying the user, the command center 440 can send the instruction to display the compute device presentation including the key indicator to the compute device 410.

In some embodiments, the cartridge 430 and the vaporizer pen 420 are configured such that, when the cartridge 430 is engaged with the vaporizer pen 420 to form a vaporizer device 401 and the cartridge 430 has been activated, when a user sucks, or "draws," on the mouthpiece, the resulting change in pressure within the vaporizer device is measured by the draw sensor 423. In response to the draw sensor 423 sensing a change in pressure (e.g., above a threshold change in pressure or to a threshold pressure level), the processor 425 of the vaporizer pen 420 can communicate the change in pressure to the processor 432 of the cartridge 430 (e.g., via the interface of the vaporizer pen 420). The processor 432 of the cartridge 430 can then actuate the heating assembly 434 to pass a current through the heating element that is in contact with, or in sufficiently close proximity to, the carrier material or a wick material containing at least a portion of the carrier material, so as to cause the volatilization of a portion of the carrier material. One or more characteristics of the current or affecting the delivery of the current passed through the heating element (e.g., voltage, wattage) can be controlled by the processor 432 based on, for example, an ambient temperature measured by a temperature sensor of the sensor(s) 461, a resistance of the heating element, and/or a heating profile or target temperature range associated with the carrier material (e.g., as determined by the processor 432 and/or provided to the processor 432 and/or stored in the memory 437 prior to use).

The volatilized carrier material, or vapor, travels toward the mouthpiece via one or more of the expansion chamber(s) and/or one or more of the fluidic channels until it exits the mouthpiece opening vaporizer for inhalation by the user. In some embodiments, the cartridge 430 and/or the vaporizer pen 420 can be coupled to the compute device 410 (e.g., a mobile phone, tablet, or computer) via, for example, Bluetooth or Wi-Fi, such that the compute device 410 can control one or more operations of the cartridge and/or the vaporizer pen 420. For example, the compute device 410 can lock and/or unlock the cartridge 430 such that the processor 432 does not actuate the heating assembly 434 when locked and the processor 432 can actuate the heating assembly 434 when unlocked.

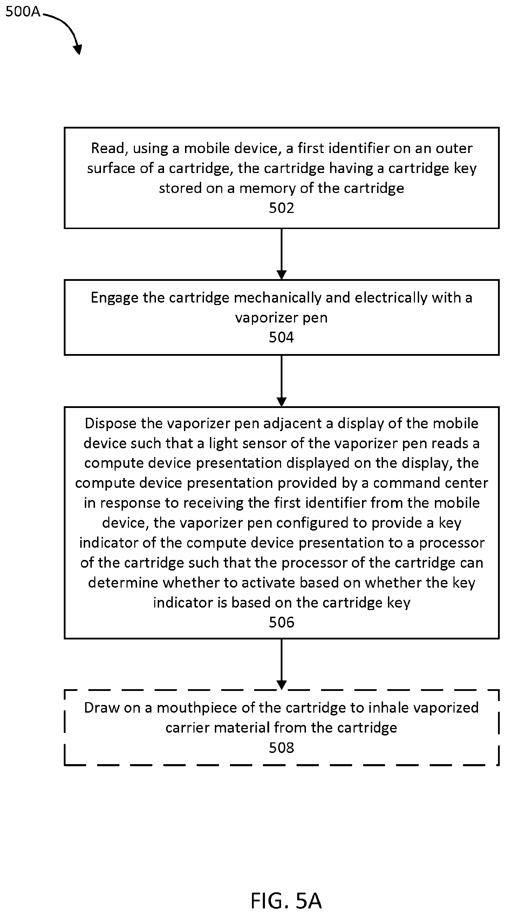

FIG. 5A illustrates a processor-implemented method 500A of managing vaporizer cartridge activation, in accordance with some embodiments. The method 500A includes reading, at 502, using a mobile device, a first identifier (e.g., a QR code) on an outer surface of a cartridge. The cartridge and the mobile device can be the same or similar in structure and/or function to any of the cartridges or compute devices described herein, such as the cartridge 430 and the compute device 410 described above. In some embodiments, the user can interact with the mobile phone to initiate a cartridge activation process (e.g., the user can select a button on a webpage to request that a command center (e.g., the command center 140 or the command center 440) provide a compute device presentation including a key indicator to the mobile device). The cartridge can be mechanically and electrically engaged, at 504, with a vaporizer pen such that a processor of the cartridge is mechanically and electrically coupled to the vaporizer pen (e.g., coupled to a draw sensor, a power supply, and/or a processor of the vaporizer pen). The vaporizer pen can be the same or similar in structure and/or function to the vaporizer pen described above. The vaporizer pen can then be disposed, at 506, adjacent a display of the mobile device such that a light sensor of the vaporizer pen can read a compute device presentation including the key indicator visually displayed on the display of the mobile device. For example, the vaporizer can be arranged such that the side of the vaporizer including the light sensor contacts a surface of the display including the compute device presentation. For example, in some embodiments, the vaporizer can have a flat elongated side surface that can be placed in contact with a display screen of the mobile device. A sensor can be disposed within or can be configured to sense through the flat surface or through an opening in the flat surface. In some embodiments, the flat surface may be a side of the vaporizer that faces toward or away from the user when the user engages a mouthpiece of the vaporizer with the user's mouth. In some embodiments, the side surface may be or may be one of the largest, longest, second largest, second longest, and/or widest surfaces of the vaporizer. The compute device presentation can include a color, a series of colors, and/or a series of flashes of a color or series of colors (e.g., arranged as a solid square or rectangle on the display). The compute device presentation can be displayed in response to the mobile device sending the first identifier to a command center that can be the same or similar in structure and/or function to the command center. The command center can provide instructions to display the compute device presentation to the mobile device for presentation in response to receiving the first identifier. The vaporizer pen can be configured to provide the key indicator or a signal or code based on the light sensor's reading of the compute device presentation to a processor of the cartridge such that the processor of the cartridge can determine whether to activate based on whether the key indicator is based on (e.g., matches or matches a derivative of) the cartridge key. If the processor of the cartridge identifies the data of the key indicator as corresponding to the cartridge key of the cartridge, the processor can activate the cartridge such that the user can draw on a mouthpiece of the cartridge to inhale vaporized carrier material from the cartridge. As shown in FIG. 5A, in some embodiments, if the cartridge has been activated, the user can optionally draw, at 508, on the mouthpiece of the cartridge to inhale vaporized carrier material from the cartridge. Although not shown in FIG. 5A, the method 500A can include any of the activation steps and/or conditions described above with respect to the system 100 or the system 400, such as the steps associated with the challenge-response process and/or the steps associated with the calibration sequence.

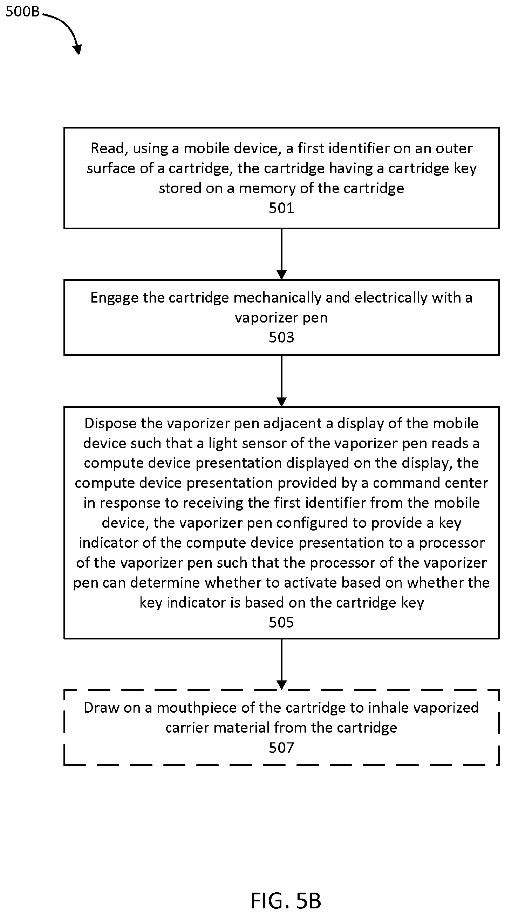

FIG. 5B illustrates a processor-implemented method 500B of managing vaporizer cartridge activation, in accordance with some embodiments. The method 500B includes reading, at 501, using a mobile device, a first identifier (e.g., a QR code) on an outer surface of a cartridge. The cartridge and the mobile device can be the same or similar in structure and/or function to any of the cartridges or compute devices described herein, such as the cartridge 430 and the compute device 410 described above. In some embodiments, the user can interact with the mobile phone to initiate a cartridge activation process (e.g., the user can select a button on a webpage to request that a command center (e.g., the command center 140 or the command center 440) provide a compute device presentation including a key indicator to the mobile device). The cartridge can be mechanically and electrically engaged, at 503, with a vaporizer pen such that a processor of the cartridge is mechanically and electrically coupled to the vaporizer pen (e.g., coupled to a draw sensor, a power supply, and/or a processor of the vaporizer pen). The vaporizer pen can be the same or similar in structure and/or function to the vaporizer pen described above. The vaporizer pen can then be disposed, at 505, adjacent a display of the mobile device such that a light sensor of the vaporizer pen can read a compute device presentation including the key indicator visually displayed on the display of the mobile device. For example, the vaporizer can be arranged such that the side of the vaporizer including the light sensor contacts a surface of the display including the compute device presentation. The compute device presentation can include a color, a series of colors, and/or a series of flashes of a color or series of colors (e.g., arranged as a solid square or rectangle on the display). The compute device presentation can be displayed in response to the mobile device sending the first identifier to a command center that can be the same or similar in structure and/or function to the command center. The command center can provide instructions to display the compute device presentation to the mobile device for presentation in response to receiving the first identifier. A processor of the vaporizer pen can be configured to receive the key indicator or a signal or code based on the light sensor's reading of the compute device presentation and to determine whether to activate based on whether the key indicator is based on (e.g., matches or matches a derivative of) the cartridge key. If the processor of the vaporizer pen identifies the data of the key indicator as corresponding to the cartridge key of the cartridge, the processor can activate such that the user can draw on a mouthpiece of the cartridge to inhale vaporized carrier material from the cartridge. As shown in FIG. 5A, in some embodiments, if the cartridge has been activated, the user can optionally draw, at 507, on the mouthpiece of the cartridge to inhale vaporized carrier material from the cartridge. Although not shown in FIG. 5B, the method 500B can include any of the activation steps and/or conditions described above with respect to the system 100 or the system 400, such as the steps associated with the challenge-response process and/or the steps associated with the calibration sequence. In some embodiments, rather requesting activation of a cartridge, activation of a vaporizer device or a pen portion of a vaporizer device can be requested based on one or more of the cartridge identifier, a vaporizer device identifier, a pen portion identifier, and/or any of the other identifiers or information described herein, and the processor of the vaporizer or the vaporizer pen can determining if a key indicator sensed (e.g., in an indication including in a compute device presentation) by the vaporizer or the vaporizer pen is based on a key stored on a memory of the cartridge or, alternatively, is based on a key stored on a memory of the vaporizer device or pen portion and can determine whether to activate the vaporizer or pen portion based on a determination that the key indicator is based on the key.

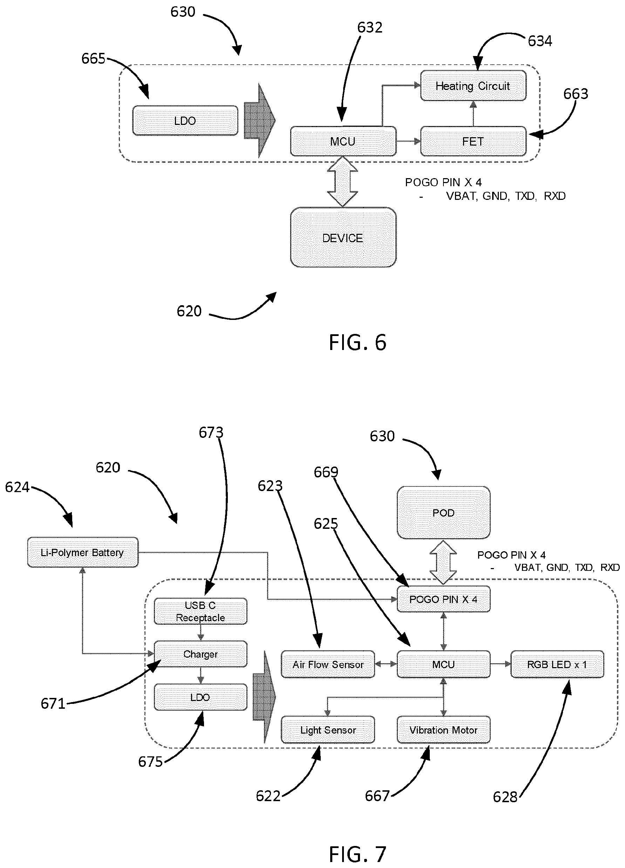

FIG. 6 is a schematic illustration of the components of a cartridge 630 couplable to a vaporizer pen 620 (referred to as a device in FIG. 4). The cartridge 630 can be the same or similar in structure and/or function to any of the cartridges described herein, such as the cartridge 430. As shown, the cartridge 630 can include a microcontroller (MCU) 632 that can be the same or similar in structure and/or function to a combination of the processor 432 and memory 437 described above with respect to the cartridge 430. The cartridge 630 includes a heating circuit 634 that can be the same or similar in structure and/or function to the heating assembly 434 and/or heating circuitry of the heating assembly 434 described above with respect to the cartridge 430. The cartridge 630 includes a field-effect transistor (FET) circuit 663 and a low-dropout regulator (LDO) 665. The FET circuit 663 can control the operation of the heating circuit 634 under the control of the MCU 632. As shown, the MCU 632 can be configured to be electrically coupled to the vaporizer pen 620 for transmission of power and communication from the device via four pogo pin connectors (e.g., of the vaporizer pen 620). The MCU 632 operates using power drawn from the vaporizer pen 620 and controls the supply of power from the vaporizer pen 620 to the other components of the cartridge 630 (e.g., the heating circuit 634).

FIG. 7 is a schematic illustration of the components of the vaporizer pen 620 couplable to the cartridge 630 (referred to as a pod in FIG. 4). The vaporizer pen 620 can be the same or similar in structure and/or function to any of the vaporizer pens described herein, such as the vaporizer pen 420. As shown, the vaporizer pen 620 includes a microcontroller (MCU) 625 that can be the same or similar in structure and/or function to the processor 125 of the vaporizer pen 420. The vaporizer pen 620 includes an air flow sensor 623, a light sensor 622, a vibration motor 667 configured to be used as an indicator, and an indicator 628 (e.g., an RGB LED). Additionally, the vaporizer pen 620 also includes a power supply 624 (e.g., a Li-polymer battery) configured to provide power to the cartridge 630 via the four pogo pin connectors 669 of the vaporizer pen 620. The power supply 624 is also electrically coupled to a charger 671 that is coupled to a USB-C receptacle 673 such that the power supply 624 can be charged to increase the level of power in the power supply 624 via the USB-C 673 receptacle. The vaporizer pen 620 also includes a low-dropout regulator (LDO) 675.

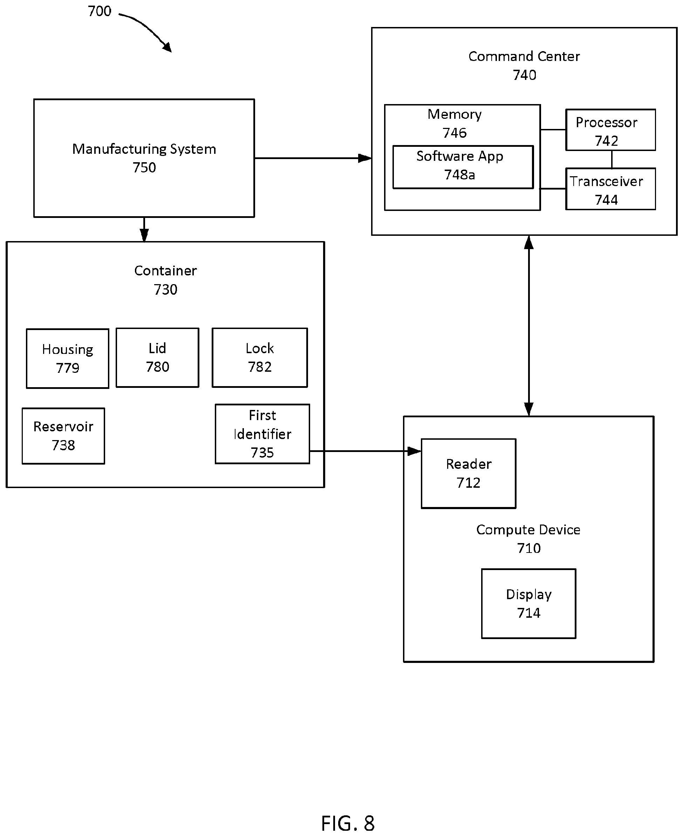

FIG. 8 is a schematic illustration of a system 700 for managing container security. The system 700 can be similar in structure and/or function to any of the systems described herein, such as the system 100 and/or the system 400. The system 700 includes a container 730 including a housing 779 and a lid 780, the housing 779 and the lid 780 defining a reservoir 738. The lid 780, container 730, and reservoir 738 can each be any suitable shape and size such that one or more objects can be securely stored in the reservoir 738. The system 700 also includes a command center 740 (e.g., a cloud-based server, a centralized server and/or the like) in wireless network communication with a manufacturing system 750 (e.g., an empty container manufacturing system, a filling system, and/or a packaging system) and a compute device 710. The command center 740, the manufacturing system 750, and the compute device 710 can be the same or similar in structure and/or function to the command center 140, the manufacturing system 150, and the compute device 110, respectively.

The container 730 can include a first identifier 735 disposed on an outer surface of the housing 779 or the lid 780. The first identifier 735 can be attached to the container 730 by the manufacturing system 750 (e.g., an empty container manufacturing system, a filling system, and/or a packaging system). The manufacturing system 750 can provide the first identifier 735 to the command center 740, and the first identifier 735 can be stored in a database stored in the memory 746 of the command center 740. The manufacturing system 750 (e.g., a fill station of the manufacturing system 750) can also provide fill data to the command center 740 such that the first identifier 735 can be associated with the fill data in the database stored in the memory 746 of the command center 740. The fill data can include, for example, identifiers of one or more of: container type, container capacity, content type, content amount, content origin/provenance, content constituent concentration(s), fill date/time stamp, fill conditions, etc.

The first identifier 735 can include any suitable data configured to identify the container 730 and representable visually on the container 730 (e.g., a serial number, a barcode, a QR code) such that the container 730 may be identified and/or recognized by an external device (e.g., a fill station of the manufacturing system 150 and/or a reader 712 of the compute device 710). The compute device 710 (e.g., a smartphone) can include a reader 712 (e.g., a camera and related software application stored on the compute device 710) configured to read the first identifier 735 (e.g., a QR code).

The lid 780 can be coupled to or include a combination lock 782. The combination lock 782 is configured to maintain the lid closed relative to the housing 779 such that the interior of the housing 779 (i.e., the reservoir) is inaccessible from outside the container 730. The combination lock 782 can be, for example, a three or four digit combination lock.

The command center 740 can include a processor 742, a transceiver 744, and a memory 746. The memory 746 of the command center 740 can store a software application ("app") 748a. In some implementations, an administrator of the command center 740 interacts with the software app 748a via an administrator view of the app, rendered via a graphical user interface (GUI) of a compute device in wireless or wired network communication therewith, and a user interacts with the software app 748a via a user view of the app, rendered via a graphical user interface (GUI) of a compute device (e.g., the display 714 of the compute device 710) of the user in wireless network communication with the command center 740. The app 748a can include one or more software modules.

In use, a user can use the compute device 710 to read (e.g. image or scan) the first identifier 735 disposed on the outer surface of the container 730. The compute device 710 can send a communication to the command center 740 based on the first identifier 735. In some embodiments, the command center 740 can then send a communication to the compute device 710 instructing the compute device 710 to present information related to the contents of the reservoir 138 (e.g., based on the fill data associated with the first identifier 735 stored in the memory 746).

The command center 740 can determine a subscription status of the user. If the subscription status is active, the command center 740 can provide an instruction to the compute device 710 to display the combination associated with the combination lock 782 on the display 714. The user can then enter the combination into the combination lock 782 to open the box and remove the lid 780 such that the contents of the container 730 can be accessed. In some embodiments, the combination lock 782 can be digital. In some embodiments, the combination lock 782 can include a keypad and display. In some embodiments, the combination lock 782 is analog.

If the subscription status is inactive (e.g., the subscription associated with the user or the compute device 710 has lapsed or has not been established), the subscription does not send the combination associated with the combination lock 782 to the compute device 710. The command center 740 can send an instruction to the compute device 710 to display a notification regarding the subscription status of the user or the compute device 710 on the display 714. The command center 740 can send an instruction to the compute device 710 for the compute device 710 to prompt the user to change the subscription status to active (e.g., via presenting a webpage for adding funds and/or creating an active subscription). Once the user has taken the steps required to change the subscription status stored in the command center 740 to active (e.g., via engagement with the compute device 710), the compute device 710 can communicate the instructions to display the combination on the display 714 of the compute device 710 to the compute device 710.

In some embodiments, alternatively or in addition to the display the combination being provided to the compute device 710 for display in response to the command center 740 determining that the first identifier 735 is associated with a particular subscription status condition, the combination can be provided to the compute device 710 for display upon the command center 740 determining that the first identifier 735 is associated with any suitable condition, such as, for example, if the first identifier 735 is associated with certain conditions such as a prescription status condition, a non-recall condition, a registration condition, or a time-based condition as described herein.

Although not shown, in some embodiments, instead of or in addition to the combination lock 782, the container 730 can include a processor, a memory, a light sensor (e.g., an RGB sensor), and a lock 782 configured to be transitioned between a locked and an unlocked configuration by the processor. A container key (also referred to as a cartridge key) can be stored in the memory of the container 730, and can be the same or similar in structure and/or function to the second identifier described above with respect to the system 100. The command center 740 can be configured to send an instruction to the compute device 710 to display a compute device presentation including a key indicator on the display 714. The compute device presentation can be the same or similar in structure and/or function to the compute device presentation described above with respect to the system 100. For example, the compute device presentation can include one or more colors or series of colors or flashes of color(s) configured to be displayed on the display 714 and configured to be read by the light sensor. The light sensor can be activated (e.g., via pressing an associated button of the container 730) to read the display 714 in the same manner or similarly as described above with respect to the light sensor 122 above. The processor of the container 730 can determine if the key indicator corresponds to the container key stored in the memory of the container 730. If the key indicator corresponds to the container key, the processor can send an instruction for the lock 782 to open. If the key indicator does not correspond to the container key, the processor does not send an instruction for the lock 782 to open.

In some embodiments, alternatively or in addition to the compute device presentation including the key indicator being provided to the compute device 710 in response to the command center 740 determining that the first identifier 735 is associated with a particular subscription status condition, the computer device presentation including the key indicator can be provided to the compute device 710 upon the command center 740 determining that the first identifier 735 is associated with any suitable condition, such as, for example, if the first identifier 735 is associated with certain conditions such as a prescription status condition, a non-recall condition, a registration condition, or a time-based condition as described herein.

Although not shown, in some embodiments, instead of or in addition to the combination lock 782, the container 730 can include a processor, a memory, an antenna or transceiver component, and a lock configured to be transitioned between a locked and an unlocked configuration by the processor based on wireless communication received by the antenna or transceiver component. For example, the antenna or transceiver component can be configured for wireless (e.g., Bluetooth or WAN) communication with the command center 740 and/or other remote compute device (such as the compute device 710). In response to the compute device 710 transmitting the first identifier 735 to the command center 740, the command center 740 can send an unlock instruction directly to the antenna or transceiver component (e.g., if the first identifier 735 is associated with certain conditions such as a subscription status condition, a prescription status condition, a non-recall condition, a registration condition, or a time-based condition as described herein).

In some embodiments, the container 730 can include one or more features having dosing or partial dispensing capabilities. For example, the container can be configured to dispense or allow access to only a predetermined portion of the contents of the container 730 per "unlock" or per provided combination. In some embodiments, the container 730 may have distinct cavities, with each cavity accessible via a distinct combination or via a distinct cartridge key. In some embodiments, only one cavity may be accessible per a predetermined time period or duration. In some embodiments, the container 730 may include a dispensing component that is configured to move a predetermined portion of the contents of the container 730 toward an accessible cavity of the container 730 per "unlock" or per provided combination.

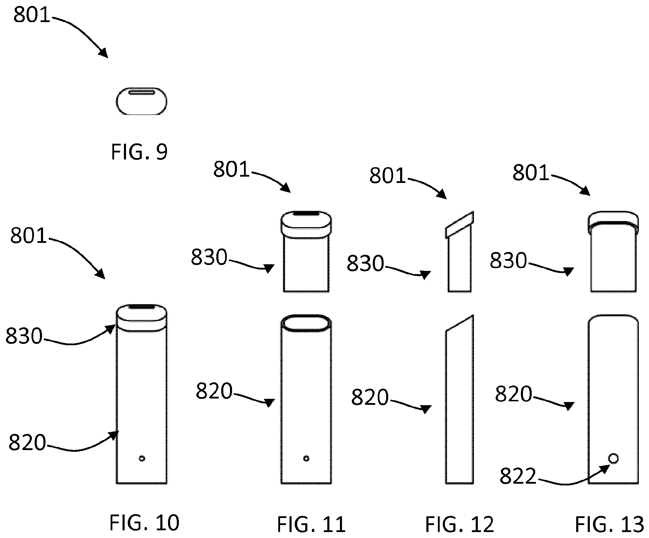

FIGS. 9-13 are various views of a vaporizer 801. The vaporizer 801 can be similar in structure and/or function to any of the devices described herein, such as the device 101 and/or the vaporizer device 401. The vaporizer 801 can include a cartridge 830 and a vaporizer pen 820. The cartridge 830 and the vaporizer pen 820 can be the same or similar in structure and/or function to any of the cartridges and vaporizer pens described herein. For example, the vaporizer pen 820 includes a light sensor 822. FIG. 9 is a top view of the vaporizer 801 of the vaporizer in an assembled configuration. FIG. 10 is a front view of the vaporizer 801 in an assembled configuration. FIGS. 11, 12, and 13 are a front view, a side view, and a back view of the vaporizer 801 with the cartridge 830 separated from the vaporizer pen 820.

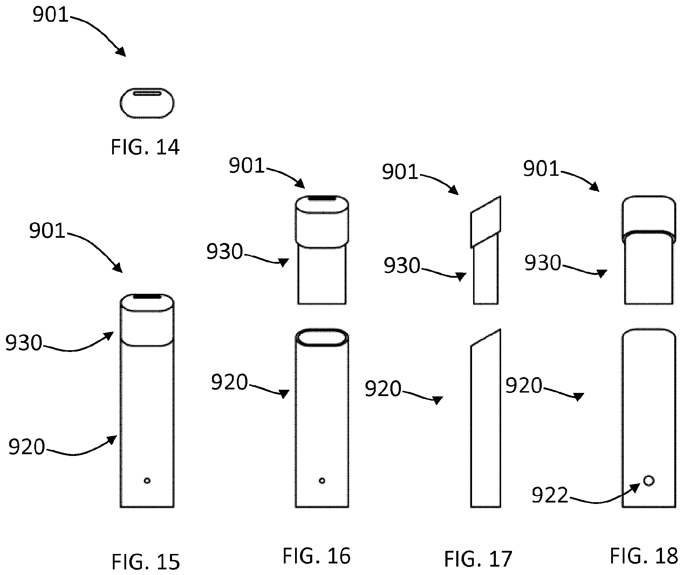

FIGS. 14-18 are various views of a vaporizer 901. The vaporizer 901 can be similar in structure and/or function to any of the devices described herein, such as the device 101 and/or the vaporizer device 401. The vaporizer 901 can include a cartridge 930 and a vaporizer pen 920. The cartridge 930 and the vaporizer pen 920 can be the same or similar in structure and/or function to any of the cartridges and vaporizer pens described herein. For example, the vaporizer pen 920 includes a light sensor 922. FIG. 14 is a top view of the vaporizer 901 of the vaporizer in an assembled configuration. FIG. 15 is a front view of the vaporizer 901 in an assembled configuration. FIGS. 16, 17, and 18 are a front view, a side view, and a back view of the vaporizer 901 with the cartridge 930 separated from the vaporizer pen 920.

Although some of the embodiments described above are described as being directed toward the activation and/or unlocking of a vaporizer device, in some embodiments, the activation-related components and methods described above may be used to activate any suitable dispensing device for dispensing of, for example, one or more metered doses of a substance.

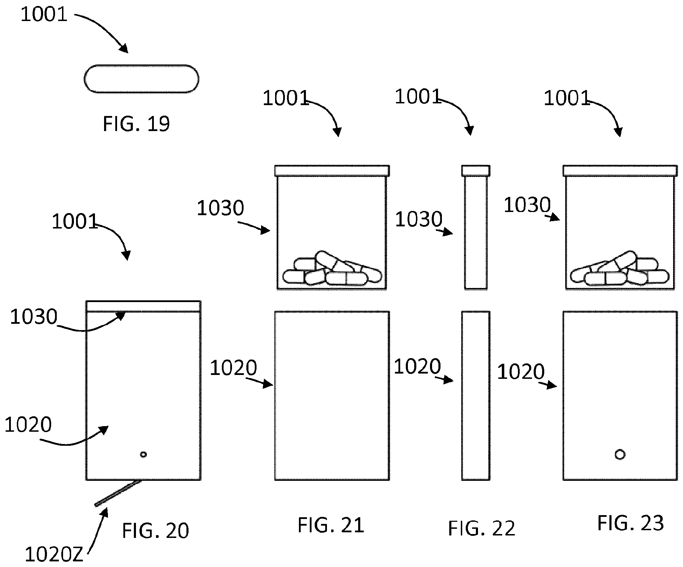

For example, FIGS. 19-23 are various views of a capsule dispensing system 1001. The capsule dispensing system 1001 can be similar in structure and/or function to any of the devices described herein, such as the device 101 and/or the vaporizer device 401. The capsule dispensing system 1001 includes a cartridge 1030 and a dispensing device 1020. The cartridge 1030 can contain any suitable amount of medication (e.g., pills or tablets). The dispensing device 1020 can be configured to release a predetermined amount of the medication from the cartridge 1030 per interaction and/or per a predetermined period of time (e.g., via a door 1020Z). The cartridge 1030 and the dispensing device 1020 can be the same or similar in structure and/or function to any of the cartridges and vaporizer pens, respectively, and/or containers described herein. For example, in some embodiments, the cartridge 1030 can include a first identifier that is the same or similar in structure and/or function to any of the first identifiers described herein and can include a cartridge key stored in a memory of the cartridge 1030 that is the same or similar in structure and/or function to any of the cartridge keys described herein. A compute device such as any of the compute devices described herein can be configured to read the first identifier and transmit the first identifier to a command center such as any of the command centers described herein. In response to the first identifier being associated with one or more conditions (e.g., a subscription condition, a time-based conditions, a non-recall condition, and/or a prescription condition), the command center can transmit an instruction to the compute device to present a compute device presentation on a display of the compute device. The compute device presentation can be the same or similar as any compute device presentation described herein. For example, the compute device presentation can include a key indicator. The dispensing device 1020 can include a light sensor configured to read the compute device presentation and a processor configured to transmit the key indicator to a processor of the cartridge 1030. The processor of the cartridge 1030 can be configured to determine whether to allow administration of the enclosed medication based on whether the key indicator is based on the cartridge key. If so, the dispensing device 1020 can be configured to dispense a predetermined amount of the medication from the cartridge 1030 to the user (e.g., via opening a door). In some embodiments, the compute device presentation can include the predetermined amount of the medication and any other suitable dosage parameters (e.g., time and amount-based parameters).

In some embodiments, the dispensing device 1020 and/or the cartridge 1030 can include an anti-tampering material that, if broken, can ruin and/or disable the medication in the cartridge 1030 or the dispensing device 1020. In some embodiments, the dispensing device 1020 and/or the cartridge 1030 can be formed of durable materials that are resistant to tampering. In some embodiments, the compute device and/or the dispensing device 1020 can include a fingerprint sensor and be configured such that a user's fingerprint must be successfully scanned prior to activating or operating the dispensing device 1020 or the cartridge 1030. In some embodiments, rather than the cartridge 1030 comparing a cartridge key with the key indicator to determine whether to activate the cartridge 1030, the compute device presentation can include instructions for the dispensing device to initiate dispensing of the medication from the cartridge 1030 based, at least in part, on the first identifier.

In some embodiments, a user may receive a notification from the command center via the compute device that a dose is available. The dispensing device 1020 can then be used to dispense a predetermined dose of the medication from the cartridge 1030 to the user (e.g., upon affirmative interaction of the user with the compute device or with a button of the dispensing device 1020). For example, the notification may include an interaction feature. The user can engage with the interaction feature to initiate the display of a compute device presentation that is the same or similar in structure and/or function to any of the compute device presentations described herein). The user can dispose the dispensing device 1020 adjacent the display such that the light sensor of the dispensing device 1020 reads the compute device presentation. In response to the code read from the compute device presentation by the light sensor, a processor of the dispensing device 1020 can unlock a dose of a medication disposed in the dispensing device 1020 or the cartridge 1030 such that the dose can be dispensed from the dispensing device 1020. The dispensing device 1020 can then lock and remain dormant until a new dose is available (as determined by the command center) and the unlocking process is initiated by the user.

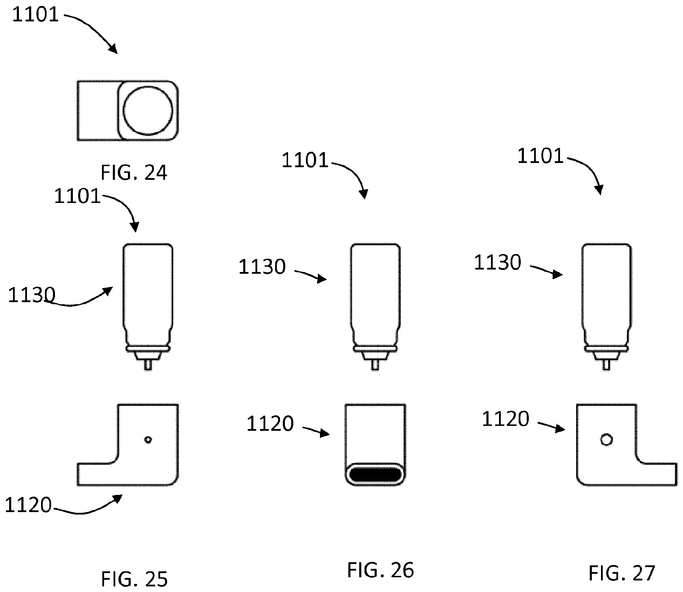

FIGS. 24-27 are various views of an inhaler device 1101. The inhaler device 1101 can be similar in structure and/or function to any of the devices described herein, such as the device 101 and/or the vaporizer device 401. The capsule dispensing system 1101 includes a cartridge 1130 and a dispensing device 1120. The cartridge 1130 can contain any suitable amount of medication (e.g., pressurized liquid and/or gaseous medication for inhalation). The dispensing device 1120 can be configured to release a predetermined metered amount of the medication from the cartridge 1130 per interaction and/or per a predetermined period of time (e.g., via a mouthpiece opening). The cartridge 1130 and the dispensing device 1120 can be the same or similar in structure and/or function to any of the cartridges and vaporizer pens, respectively, and/or containers described herein. For example, in some embodiments, the cartridge 1130 can include a first identifier that is the same or similar in structure and/or function to any of the first identifiers described herein and can include a cartridge key stored in a memory of the cartridge 1130 that is the same or similar in structure and/or function to any of the cartridge keys described herein. A compute device such as any of the compute devices described herein can be configured to read the first identifier and transmit the first identifier to a command center such as any of the command centers described herein. In response to the first identifier being associated with one or more conditions (e.g., a subscription condition, a time-based condition, a non-recall condition, and/or a prescription condition), the command center can transmit an instruction to the compute device to present a compute device presentation on a display of the compute device. The compute device presentation can be the same or similar as any compute device presentation described herein. For example, the compute device presentation can include a key indicator. The dispensing device 1120 can include a light sensor configured to read the compute device presentation and a processor configured to transmit the key indicator to a processor of the cartridge 1130. The processor of the cartridge 1130 can be configured to determine whether to allow administration of the enclosed medication based on whether the key indicator is based on the cartridge key. If so, the dispensing device 1120 can be configured to dispense a predetermined amount of the medication from the cartridge 1130 to the user (e.g., via allowing triggering of an administration trigger of the dispensing device 1120). In some embodiments, the compute device presentation can include the predetermined amount of the medication and any other suitable dosage parameters (e.g., time and amount-based parameters).

In some embodiments, the dispensing device 1120 and/or the cartridge 1130 can include an anti-tampering material that, if broken, can ruin and/or disable the medication in the cartridge 1130 or the dispensing device 1120. In some embodiments, the dispensing device 1120 and/or the cartridge 1130 can be formed of durable materials that are resistant to tampering. In some embodiments, the compute device and/or the dispensing device 1120 can include a biometric sensor such as any of the biometric sensors described herein (e.g., a fingerprint sensor) and be configured such that a biometric characteristic of the user (e.g., a user's fingerprint) must be successfully scanned prior to activating or operating the dispensing device 1120 or the cartridge 1130. In some embodiments, rather than the cartridge 1130 comparing a cartridge key with the key indicator to determine whether to activate the cartridge 1130, the compute device presentation can include instructions for the dispensing device to initiate dispensing of the medication from the cartridge 1130 based, at least in part, on the first identifier.

In some embodiments, a user may receive a notification from the command center via the compute device that a dose is available. The dispensing device 1120 can then be used to dispense a predetermined dose of the medication from the cartridge 1130 to the user (e.g., upon affirmative interaction of the user with the compute device or with a button of the dispensing device 1120). For example, the notification may include an interaction feature. The user can engage with the interaction feature to initiate the display of a compute device presentation that is the same or similar in structure and/or function to any of the compute device presentations described herein). The user can dispose the dispensing device 1120 adjacent the display such that the light sensor of the dispensing device 1120 reads the compute device presentation. In response to the code read from the compute device presentation by the light sensor, a processor of the dispensing device 1120 can unlock a dose of a medication disposed in the dispensing device 1120 or the cartridge 1130 such that the dose can be dispensed from the dispensing device 1120. The dispensing device 1120 can then lock and remain dormant until a new dose is available (as determined by the command center) and the unlocking process is initiated by the user.

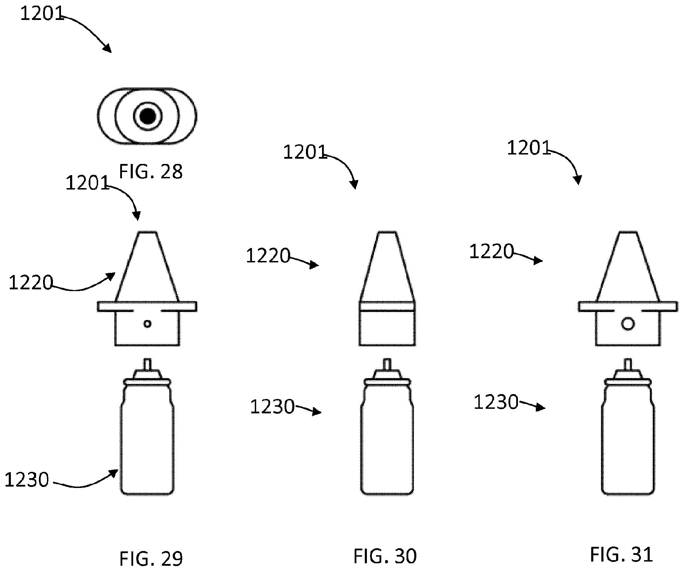

FIGS. 28-31 are various views of a nasal drug delivery device 1201. The nasal drug delivery device 1201 can be similar in structure and/or function to any of the devices described herein, such as the device 101 and/or the vaporizer device 401. The capsule dispensing system 1201 includes a cartridge 1230 and a dispensing device 1220. The cartridge 1230 can contain any suitable amount of medication (e.g., pressurized liquid and/or gaseous medication for inhalation). The dispensing device 1220 can be configured to release a predetermined metered amount of the medication from the cartridge 1230 from a nozzle of the dispensing device 1220 per interaction and/or per a predetermined period of time. The cartridge 1230 and the dispensing device 1220 can be the same or similar in structure and/or function to any of the cartridges and vaporizer pens, respectively, and/or containers described herein. For example, in some embodiments, the cartridge 1230 can include a first identifier that is the same or similar in structure and/or function to any of the first identifiers described herein and can include a cartridge key stored in a memory of the cartridge 1230 that is the same or similar in structure and/or function to any of the cartridge keys described herein. A compute device such as any of the compute devices described herein can be configured to read the first identifier and transmit the first identifier to a command center such as any of the command centers described herein. In response to the first identifier being associated with one or more conditions (e.g., a subscription condition, a time-based conditions, a non-recall condition, and/or a prescription condition), the command center can transmit an instruction to the compute device to present a compute device presentation on a display of the compute device. The compute device presentation can be the same or similar as any compute device presentation described herein. For example, the compute device presentation can include a key indicator. The dispensing device 1220 can include a light sensor configured to read the compute device presentation and a processor configured to transmit the key indicator to a processor of the cartridge 1230. The processor of the cartridge 1230 can be configured to determine whether to allow administration of the enclosed medication based on whether the key indicator is based on the cartridge key. If so, the dispensing device 1220 can be configured to dispense a predetermined amount of the medication from the cartridge 1230 to the user (e.g., via allowing triggering of an administration trigger of the dispensing device 1220). In some embodiments, the compute device presentation can include the predetermined amount of the medication and any other suitable dosage parameters (e.g., time and amount based parameters).

In some embodiments, the dispensing device 1220 and/or the cartridge 1230 can include an anti-tampering material that, if broken, can ruin and/or disable the medication in the cartridge 1230 or the dispensing device 1220. In some embodiments, the dispensing device 1220 and/or the cartridge 1230 can be formed of durable materials that are resistant to tampering. In some embodiments, the compute device and/or the dispensing device 1220 can include a fingerprint sensor and be configured such that a user's fingerprint must be successfully scanned prior to activating or operating the dispensing device 1220 or the cartridge 1230. In some embodiments, rather than the cartridge 1230 comparing a cartridge key with the key indicator to determine whether to activate the cartridge 1230, the compute device presentation can include instructions for the dispensing device to initiate dispensing of the medication from the cartridge 1230 based, at least in part, on the first identifier.

In some embodiments, a user may receive a notification from the command center via the compute device that a dose is available. The dispensing device 1220 can then be used to dispense a predetermined dose of the medication from the cartridge 1230 to the user (e.g., upon affirmative interaction of the user with the compute device or with a button of the dispensing device 1220). For example, the notification may include an interaction feature. The user can engage with the interaction feature to initiate the display of a compute device presentation that is the same or similar in structure and/or function to any of the compute device presentations described herein). The user can dispose the dispensing device 1220 adjacent the display such that the light sensor of the dispensing device 1220 reads the compute device presentation. In response to the code read from the compute device presentation by the light sensor, a processor of the dispensing device 1220 can unlock a dose of a medication disposed in the dispensing device 1220 or the cartridge 1230 such that the dose can be dispensed from the dispensing device 1220 (e.g., via dispensing into a user's nasal opening). The dispensing device 1220 can then lock and remain dormant until a new dose is available (as determined by the command center) and the unlocking process is initiated by the user.

In some embodiments, a system, such as any of the systems described herein can be used to control activation and/or operation of a device using WAN (e.g., LTE or 3G). For example, a vaporizer pen can include an WAN-enabled chip, an antenna, and/or a GPS. The vaporizer pen could be configured to communicate with a compute device, such as any of the compute devices described herein, via a server. The vaporizer pen could be controlled (e.g., activated or unlocked) via communication with a command center, such as any of the command centers described herein, and/or by a compute device, such as any of the compute devices described herein. Additionally, the vaporizer pen can only be permitted to operate under certain conditions, such as any of the age-based, location-based, prescription-based, or other conditions described herein, as monitored and controlled by the command center and/or the compute device.

In some embodiments, the memory can also store instructions to cause the processor to determine whether the capsule is authentic based on whether the capsule identifier includes a digital signature associated with a public key stored in the memory.

In some embodiments, the memory can also store instructions to cause the processor to activate the heater control circuitry in response to the processor determining that the capsule is authentic.

In some embodiments, the memory can also store instructions to cause the processor to write a recall identifier on the capsule memory in response to the processor receiving an indication that the capsule identifier is associated with a recall via the transmitter.

In some embodiments, the memory can also store instructions to cause the processor to send, via the transmitter, a capsule attach event detection message in response to the capsule being coupled to the interface, and to activate the heater control circuitry only after receiving an unlock signal from the remote compute device.

The term "automatically" is used herein to modify actions that occur without direct input or prompting by an external source such as a user. Automatically occurring actions can occur periodically, sporadically, in response to a detected event (e.g., a user logging in), or according to a predetermined schedule.

The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may comprise a single computer-readable statement or many computer-readable statements.

Some embodiments described herein relate to a computer storage product with a non-transitory computer-readable medium (also can be referred to as a non-transitory processor-readable medium) having instructions or computer code thereon for performing various computer-implemented operations. The computer-readable medium (or processor-readable medium) is non-transitory in the sense that it does not include transitory propagating signals per se (e.g., a propagating electromagnetic wave carrying information on a transmission medium such as space or a cable). The media and computer code (also can be referred to as code) may be those designed and constructed for the specific purpose or purposes. Examples of non-transitory computer-readable media include, but are not limited to, magnetic storage media such as hard disks, floppy disks, and magnetic tape; optical storage media such as Compact Disc/Digital Video Discs (CD/DVDs), Compact Disc-Read Only Memories (CD-ROMs), and holographic devices; magneto-optical storage media such as optical disks; carrier wave signal processing modules; and hardware devices that are specially configured to store and execute program code, such as Application-Specific Integrated Circuits (ASICs), Programmable Logic Devices (PLDs), Read-Only Memory (ROM) and Random-Access Memory (RAM) devices. Other embodiments described herein relate to a computer program product, which can include, for example, the instructions and/or computer code discussed herein.

Some embodiments and/or methods described herein can be performed by software (executed on hardware), hardware, or a combination thereof. Hardware modules may include, for example, a general-purpose processor, a field programmable gate array (FPGA), and/or an application specific integrated circuit (ASIC). Software modules (executed on hardware) can be expressed in a variety of software languages (e.g., computer code), including C, C++, Java^{™}, Ruby, Visual Basic^{™}, and/or other object-oriented, procedural, or other programming language and development tools. Examples of computer code include, but are not limited to, micro-code or micro-instructions, machine instructions, such as produced by a compiler, code used to produce a web service, and files containing higher-level instructions that are executed by a computer using an interpreter. For example, embodiments may be implemented using imperative programming languages (e.g., C, Fortran, etc.), functional programming languages (Haskell, Erlang, etc.), logical programming languages (e.g., Prolog), object-oriented programming languages (e.g., Java, C++, etc.) or other suitable programming languages and/or development tools. Additional examples of computer code include, but are not limited to, control signals, encrypted code, and compressed code.

Various concepts may be embodied as one or more methods, of which at least one example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments. Put differently, it is to be understood that such features may not necessarily be limited to a particular order of execution, but rather, any number of threads, processes, services, servers, and/or the like that may execute serially, asynchronously, concurrently, in parallel, simultaneously, synchronously, and/or the like in a manner consistent with the disclosure. As such, some of these features may be mutually contradictory, in that they cannot be simultaneously present in a single embodiment. Similarly, some features are applicable to one aspect of the innovations, and inapplicable to others.

In addition, the disclosure may include other innovations not presently described. Applicant reserves all rights in such innovations, including the right to embodiment such innovations, file additional applications, continuations, continuations-in-part, divisionals, and/or the like thereof. As such, it should be understood that advantages, embodiments, examples, functional, features, logical, operational, organizational, structural, topological, and/or other aspects of the disclosure are not to be considered limitations on the disclosure as defined by the embodiments or limitations on equivalents to the embodiments. Depending on the particular desires and/or characteristics of an individual and/or enterprise user, database configuration and/or relational model, data type, data transmission and/or network framework, syntax structure, and/or the like, various embodiments of the technology disclosed herein may be implemented in a manner that enables a great deal of flexibility and customization as described herein.

All definitions, as defined and used herein, should be understood to control over dictionary definitions and/or ordinary meanings of the defined terms.

As used herein, in particular embodiments, the terms "about" or "approximately" when preceding a numerical value indicates the value plus or minus a range of 10%. Where a range of values is provided, it is understood that each intervening value, to the tenth of the unit of the lower limit unless the context clearly dictates otherwise, between the upper and lower limit of that range and any other stated or intervening value in that stated range is encompassed within the disclosure. That the upper and lower limits of these smaller ranges can independently be included in the smaller ranges is also encompassed within the disclosure, subject to any specifically excluded limit in the stated range. Where the stated range includes one or both of the limits, ranges excluding either or both of those included limits are also included in the disclosure.

The indefinite articles "a" and "an," as used herein in the specification and in the embodiments, unless clearly indicated to the contrary, should be understood to mean "at least one."

The phrase "and/or," as used herein in the specification and in the embodiments, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the embodiments, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the embodiments, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of." "Consisting essentially of," when used in the embodiments, shall have its ordinary meaning as used in the field of patent law.

As used herein in the specification and in the embodiments, the phrase "at least one," in reference to a list of one or more elements, should be understood to mean at least one element selected from any one or more of the elements in the list of elements, but not necessarily including at least one of each and every element specifically listed within the list of elements. Thus, as a non-limiting example, "at least one of A and B" (or, equivalently, "at least one of A or B," or, equivalently "at least one of A and/or B") can refer, in one embodiment, to at least one, optionally including more than one, A, with no B present; in another embodiment, to at least one, optionally including more than one, B, with no A present; in yet another embodiment, to at least one, optionally including more than one, A, and at least one, optionally including more than one, B; etc.

In the embodiments, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of' shall be closed or semi-closed transitional phrases, respectively, as set forth in the United States Patent Office Manual of Patent Examining Procedures, Section 2111.03.

While specific embodiments of the present disclosure have been outlined above, many alternatives, modifications, and variations will be apparent to those skilled in the art. Accordingly, the embodiments set forth herein are intended to be illustrative, not limiting. Various changes may be made without departing from the scope of the disclosure. Where methods and steps described above indicate certain events occurring in a certain order, those of ordinary skill in the art having the benefit of this disclosure would recognize that the ordering of certain steps may be modified and such modification are in accordance with the variations of the invention. Additionally, certain of the steps may be performed concurrently in a parallel process when possible, as well as performed sequentially as described above. The embodiments have been particularly shown and described, but it will be understood that various changes in form and details may be made.

## Claims

1. A cartridge (430), associated with a cartridge key, the cartridge (430) comprising:
a memory (437),
a cartridge processor (432) including the cartridge key stored in the memory,
and a heating assembly (434),
wherein the cartridge is configured to be electrically coupled to a vaporizer pen (420) comprising a power supply (424), such that:
the heating assembly (434) of the cartridge can operate based on power provided by the power supply in response to activation of the heating assembly; and
a key indicator can be transmitted by the vaporizer pen to the cartridge processor;
the cartridge processor (432) is configured to: compare the cartridge key stored in the memory to the key indicator, and activate the heating assembly based, at least in part, on whether the key indicator, transmitted by the vaporizer pen, is based on the cartridge key.

2. A system (400), comprising:
a vaporizer pen (420) including a power supply (424), a sensor (422) configured to sense a calibration sequence and an indication generated by a compute device (410), a memory (429), and a pen processor (425) operatively coupled to the memory and configured to identify a key indicator based on the calibration sequence and the indication sensed by the sensor; and
a cartridge according to claim 1, the cartridge configured to be electrically coupled to the vaporizer pen such that the heating assembly can operate based on power provided by the power supply and such that the key indicator can be transmitted to the cartridge processor, the cartridge processor configured to determine, whether the key indicator is based on the cartridge key, whether to allow activation of the cartridge such that the heating assembly can operate.

3. The system of claim 2, wherein the vaporizer pen (420) further comprises a housing defining an opening, the sensor being at least partially disposed within the opening such that the sensor is configured to be placed adjacent to a display (414) of the compute device when the housing is disposed adjacent to the display of the compute device.

4. The system (400) of claim 2, wherein:
the cartridge key is stored in the memory (437) of the cartridge,
the pen processor (425) is configured to request a challenge value from the cartridge processor,
in response to receiving a request from the pen processor, the cartridge processor is configured to send a challenge value to the pen processor,
in response to receiving the challenge value from the cartridge processor, the pen processor is configured to transmit a response to the cartridge processor based on the challenge value and the key indicator, and
the cartridge processor is configured to allow the heating assembly to activate in response to determining that:
the response is based in part on the challenge value,
the response is based in part on the key indicator, and
the key indicator is associated with the cartridge key.

5. The system (400) of claim 4, wherein the response to the cartridge processor includes a hash based on the challenge value and the key indicator.

6. The system (400) of claim 2, wherein the pen processor (425) is configured to identify a delimiter generated by the compute device at least one of before generating the calibration sequence or after generating the indication.

7. The system (400) of claim 2, wherein the sensor (422) is a light sensor and the indication is a visual indication, the calibration sequence and the indication being included in a visual presentation generated on a display (414) of the compute device.

8. The system (400) of claim 7, wherein the calibration sequence includes a predetermined sequence of colors and the visual indication includes a sequence of visual images including a plurality of colors, the pen processor configured to recognize the predetermined sequence of colors included in the calibration sequence, the predetermined sequence of colors of the calibration sequence including each color from the plurality of colors included in the sequence of visual images of the visual indication.

9. A method, comprising:
requesting, via interaction with a user interface (414) of a compute device (410), activation of a cartridge (430) associated with a cartridge key and including a heating assembly (434) and a cartridge processor (432) including the cartridge key stored in a memory for operation with a vaporizer pen (420) including a power supply (424) configured to provide operational power to the heating assembly;
engaging the cartridge with the vaporizer pen; and
disposing the vaporizer pen adjacent to the compute device such that a sensor (422) of the vaporizer pen can sense a calibration sequence and an indication generated by the compute device and a processor (425) of the vaporizer pen can identify a key indicator based on the calibration sequence and the indication sensed by the sensor, the cartridge processor (432) is configured to compare the cartridge key to the key indicator and determine whether to activate the heating assembly (434) based on whether the key indicator is based on the cartridge key.

10. The method of claim 9, wherein the indication includes the key indicator, the vaporizer pen (420) configured to provide a communication to the cartridge (430) based on the key indicator such that the cartridge can determine whether or not to allow operation of the heating assembly based on whether the communication is based on the key indicator and based on whether the key indicator is associated with a cartridge key stored in a memory of the cartridge.

11. The method of claim 10, wherein the communication is based in part on a challenge value provided to the vaporizer pen (420) from the cartridge (430), and the cartridge can determine whether or not to allow operation of the heating assembly based on whether the communication is based in part on the challenge value.

12. The method of claim 9, wherein the requesting includes at least one of causing the compute device (410) to scan a first identifier associated with the cartridge (430) or manually entering a second identifier associated with the cartridge via interaction with the user interface such that the compute device transmits at least one of the first identifier or the second identifier to a command center, the indication generated by the compute device being based on instructions sent to the compute device from the command center based on at least one of the first identifier or the second identifier.

13. The method of claim 9, wherein the user interface includes a display (414), the indication is a visual indication, the sensor (422) is a light sensor, the calibration sequence and the visual indication each including a sequence of visual images including a plurality of colors, the vaporizer pen (420) is configured to recognize a predetermined sequence of colors included in the calibration sequence, and the disposing includes placing a flat surface of the vaporizer pen including an opening within which the sensor is disposed in contact with the display (414) of the compute device (410) such that the sensor is aligned with the display, each visual image of the sequence of visual images displayed on the display of the compute device having a larger area than a surface area of the flat surface of the vaporizer pen contacting the display.

## Patentansprüche

1. Kartusche (430), die einem Kartuschenschlüssel zugeordnet ist, wobei die Kartusche (430) umfasst:
einen Speicher (437),
einen Kartuschenprozessor (432), der den in dem Speicher gespeicherten Kartuschenschlüssel einschließt, und eine Heizungsanordnung (434),
wobei die Kartusche ausgebildet ist, um elektrisch mit einem Verdampferstift (420) gekoppelt zu sein, der eine Stromversorgung (424) umfasst, sodass:
die Heizungsanordnung (434) der Kartusche auf der Grundlage der Leistung betrieben werden kann, die von der Stromversorgung als Reaktion auf die Aktivierung der Heizungsanordnung bereitgestellt wird; und
ein Schlüsselindikator von dem Verdampferstift an den Kartuschenprozessor übertragen werden kann;
wobei der Kartuschenprozessor (432) ausgebildet ist zum: Vergleichen des in dem Speicher gespeicherten Kartuschenschlüssels mit dem Schlüsselindikator, und Aktivieren der Heizungsanordnung mindestens teilweise auf der Grundlage davon, ob der von dem Verdampferstift übertragene Schlüsselindikator auf dem Kartuschenschlüssel basiert.

2. System (400), umfassend:
einen Verdampferstift (420), der eine Stromversorgung (424), einen Sensor (422), der ausgebildet ist, um eine Kalibriersequenz, und eine von einer Rechenvorrichtung (410) erzeugte Anzeige zu erfassen, einen Speicher (429) und einen Stiftprozessor (425) einschließt, der funktionsfähig mit dem Speicher gekoppelt ist, und ausgebildet ist, um auf der Grundlage der Kalibriersequenz und der von dem Sensor erfassten Anzeige einen Schlüsselindikator zu identifizieren; und
eine Kartusche nach Anspruch 1, die Kartusche, die ausgebildet ist, um elektrisch mit dem Verdampferstift gekoppelt zu sein, sodass die Heizungsanordnung auf der Grundlage des von der Stromversorgung bereitgestellten Stroms betrieben werden kann und sodass der Schlüsselindikator an den Kartuschenprozessor übertragen werden kann, wobei der Kartuschenprozessor ausgebildet ist, um zu bestimmen, ob der Schlüsselindikator auf dem Kartuschenschlüssel basiert, ob die Aktivierung der Kartusche zu erlauben ist, sodass die Heizungsanordnung betrieben werden kann.

3. System nach Anspruch 2, wobei der Verdampferstift (420) weiter ein Gehäuse umfasst, das eine Öffnung definiert, wobei der Sensor mindestens teilweise innerhalb der Öffnung angeordnet ist, sodass der Sensor ausgebildet ist, um neben einem Display (414) der Rechenvorrichtung platziert zu sein, wenn das Gehäuse neben dem Display der Rechenvorrichtung angeordnet ist.

4. System (400) nach Anspruch 2, wobei:
der Kartuschenschlüssel in dem Speicher (437) der Kartusche gespeichert ist,
der Stiftprozessor (425) ausgebildet ist, um einen Challenge-Wert von dem Kartuschenprozessor anzufordern,
der Kartuschenprozessor als Reaktion auf das Empfangen einer Anforderung von dem Stiftprozessor ausgebildet ist, um einen Challenge-Wert an den Stiftprozessor zu senden,
der Stiftprozessor als Reaktion auf das Empfangen des Challenge-Werts von dem Kartuschenprozessor ausgebildet ist, um eine Antwort an den Kartuschenprozessor auf der Grundlage des Abfragewerts und des Schlüsselindikators zu übertragen, und
der Kartuschenprozessor ausgebildet ist, um zu ermöglichen, dass die Heizungsanordnung aktiviert wird als Reaktion auf das Bestimmen, dass:
die Antwort teilweise auf dem Challenge-Wert basiert,
die Antwort teilweise auf dem Schlüsselindikator basiert, und
der Schlüsselindikator dem Kartuschenschlüssel zugeordnet ist.

5. System (400) nach Anspruch 4, wobei die Antwort an den Kartuschenprozessor einen Hash auf der Grundlage des Challenge-Werts und des Schlüsselindikators einschließt.

6. System (400) nach Anspruch 2, wobei der Stiftprozessor (425) ausgebildet ist, um ein Trennzeichen zu identifizieren, das von der Rechenvorrichtung mindestens eines von vor dem Erzeugen der Kalibriersequenz oder nach dem Erzeugen der Anzeige erzeugt wird.

7. System (400) nach Anspruch 2, wobei der Sensor (422) ein Lichtsensor ist und die Anzeige eine visuelle Anzeige ist, wobei die Kalibriersequenz und die Anzeige in eine visuelle Darstellung eingeschlossen sind, die auf einem Display (414) der Rechenvorrichtung erzeugt wird.

8. System (400) nach Anspruch 7, wobei die Kalibriersequenz eine vorbestimmte Sequenz von Farben einschließt und die visuelle Anzeige eine Sequenz von visuellen Bildern einschließt, die eine Vielzahl von Farben einschließt, wobei der Stiftprozessor ausgebildet ist, um die vorbestimmte Sequenz von Farben, die in die Kalibriersequenz eingeschlossen sind, zu erkennen, die vorbestimmte Sequenz von Farben der Kalibriersequenz jede Farbe aus der Vielzahl von Farben einschließt, die in der Sequenz von visuellen Bildern der visuellen Anzeige eingeschlossen sind.

9. Verfahren, umfassend:
Anfordern, über eine Interaktion mit einer Benutzeroberfläche (414) einer Rechenvorrichtung (410), einer Aktivierung einer Kartusche (430), die einem Kartuschenschlüssel zugeordnet ist und eine Heizanordnung (434) und einen Kartuschenprozessor (432) einschließt, der den in einem Speicher gespeicherten Kartuschenschlüssel für den Betrieb mit einem Verdampferstift (420) einschließt, der eine Stromversorgung (424) einschließt, die ausgebildet ist, um der Heizungsanordnung Betriebsleistung bereitzustellen;
Ineingriffbringen der Kartusche mit dem Verdampferstift; und
Anordnen des Verdampferstifts neben der Rechenvorrichtung, sodass ein Sensor (422) des Verdampferstifts eine Kalibriersequenz erfassen kann und eine von der Rechenvorrichtung erzeugte Anzeige und ein Prozessor (425) des Verdampferstifts einen Schlüsselindikator auf der Grundlage der Kalibriersequenz und der von dem Sensor erfassten Anzeige identifizieren kann, wobei der Kartuschenprozessor (432) ausgebildet ist, um den Kartuschenschlüssel mit dem Schlüsselindikator zu vergleichen und zu bestimmen, ob die Heizungsanordnung (434) auf der Grundlage darauf aktiviert werden soll, ob der Schlüsselindikator auf dem Kartuschenschlüssel basiert.

10. Verfahren nach Anspruch 9, wobei die Anzeige den Schlüsselindikator einschließt, der Verdampferstift (420) ausgebildet ist, um auf der Grundlage des Schlüsselindikators eine Kommunikation mit der Kartusche (430) bereitzustellen, sodass die Kartusche auf der Grundlage darauf bestimmen kann, ob der Betrieb der Heizungsanordnung auf der Grundlage davon zu erlauben ist oder nicht, ob die Kommunikation auf dem Schlüsselindikator basiert und darauf basiert, ob der Schlüsselindikator einem in einem Speicher der Kartusche gespeicherten Kartuschenschlüssel zugeordnet ist.

11. Verfahren nach Anspruch 10, wobei die Kommunikation teilweise auf einem Abfragewert basiert, der dem Verdampferstift (420) von der Kartusche (430) bereitgestellt wird, und die Kartusche bestimmen kann, ob der Betrieb der Heizungsanordnung auf der Grundlage davon zu erlauben ist oder nicht, ob die Kommunikation teilweise auf dem Challenge-Wert basiert.

12. Verfahren nach Anspruch 9, wobei das Anfordern mindestens eines von dem Veranlassen der Rechenvorrichtung (410), eine erste, der Kartusche (430) zugeordnete Kennung zu scannen, oder dem manuellen Eingeben einer zweiten der Kartusche zugeordneten Kennung über eine Interaktion mit der Benutzeroberfläche einschließt, sodass die Rechenvorrichtung mindestens eine von der ersten Kennung oder der zweiten Kennung an eine Kommandozentrale überträgt, die von der Rechenvorrichtung erzeugte Anzeige auf Anweisungen basiert, die auf der Grundlage von mindestens einer der ersten Kennung oder der zweiten Kennung von der Kommandozentrale an die Rechenvorrichtung gesendet werden.

13. Verfahren nach Anspruch 9, wobei die Benutzeroberfläche ein Display (414) einschließt, die Anzeige eine visuelle Anzeige ist, der Sensor (422) ein Lichtsensor ist, die Kalibriersequenz und die visuelle Anzeige jeweils eine Sequenz visueller Bilder einschließen, die eine Vielzahl von Farben einschließen, der Verdampferstift (420) ausgebildet ist, um eine vorbestimmte Sequenz von Farben zu erkennen, die in der Kalibriersequenz eingeschlossen sind, und das Anordnen das Platzieren einer flachen Oberfläche des Verdampferstifts, die eine Öffnung einschließt, in welcher der Sensor angeordnet ist, in Kontakt mit dem Display (414) der Rechenvorrichtung (410) einschließt, dass der Sensor mit dem Display ausgerichtet ist, wobei jedes visuelle Bild der auf dem Display der Rechenvorrichtung angezeigten Sequenz von visuellen Bildern eine größere Fläche aufweist als eine Fläche der flachen Oberfläche des Verdampferstifts, welche das Display berührt.

## Revendications

1. Cartouche (430), associée à une clé de cartouche, la cartouche (430) comprenant :
une mémoire (437),
un processeur de cartouche (432) incluant la clé de cartouche stockée dans la mémoire,
et un ensemble chauffant (434),
dans laquelle la cartouche est configurée pour être couplée électriquement à un stylo vaporisateur (420) comprenant une alimentation électrique (424), de sorte que :
l'ensemble chauffant (434) de la cartouche puisse fonctionner en se basant sur la puissance fournie par l'alimentation électrique en réponse à l'activation de l'ensemble chauffant ; et
un indicateur de clé puisse être transmis par le stylo vaporisateur au processeur de cartouche ;
le processeur de cartouche (432) étant configuré pour : comparer la clé de cartouche stockée dans la mémoire à l'indicateur de clé, et activer l'ensemble chauffant en se basant, au moins en partie, sur le fait que l'indicateur de clé, transmis par le stylo vaporisateur, est basé ou non sur la clé de cartouche.

2. Système (400), comprenant :
un stylo vaporisateur (420) incluant une alimentation électrique (424), un capteur (422) configuré pour détecter une séquence d'étalonnage et une indication générée par un dispositif de calcul (410), une mémoire (429), et un processeur (425) de stylo fonctionnellement couplé à la mémoire et configuré pour identifier un indicateur de clé en se basant sur la séquence d'étalonnage et l'indication détectée par le capteur ; et
une cartouche selon la revendication 1, la cartouche étant configurée pour être couplée électriquement au stylo vaporisateur de sorte que l'ensemble chauffant puisse fonctionner en se basant sur la puissance fournie par l'alimentation électrique et de sorte que l'indicateur de clé puisse être transmis au processeur de cartouche, le processeur de cartouche étant configuré pour déterminer, selon que l'indicateur de clé est basé ou non sur la clé de cartouche, s'il convient d'autoriser l'activation de la cartouche de sorte que l'ensemble chauffant puisse fonctionner.

3. Système selon la revendication 2, dans lequel le stylo vaporisateur (420) comprend en outre un logement définissant une ouverture, le capteur étant au moins partiellement disposé à l'intérieur de l'ouverture de sorte que le capteur soit configuré pour être placé adjacent à une unité d'affichage (414) du dispositif de calcul lorsque le logement est disposé adjacent à l'unité d'affichage du dispositif de calcul.

4. Système (400) selon la revendication 2, dans lequel :
la clé de cartouche est stockée dans la mémoire (437) de la cartouche,
le processeur (425) de stylo est configuré pour demander une valeur de défi au processeur de cartouche,
en réponse à la réception d'une demande du processeur de stylo, le processeur de cartouche est configuré pour envoyer une valeur de défi au processeur de stylo,
en réponse à la réception de la valeur de défi du processeur de cartouche, le processeur de stylo est configuré pour transmettre une réponse au processeur de cartouche en se basant sur la valeur de défi et l'indicateur de clé, et
le processeur de cartouche est configuré pour permettre à l'ensemble chauffant de s'activer en réponse à la détermination que :
la réponse est basée en partie sur la valeur de défi,
la réponse est basée en partie sur l'indicateur de clé, et
l'indicateur de clé est associé à la clé de cartouche.

5. Système (400) selon la revendication 4, dans lequel la réponse au processeur de cartouche inclut une valeur de hachage basée sur la valeur de défi et l'indicateur de clé.

6. Système (400) selon la revendication 2, dans lequel le processeur (425) de stylo est configuré pour identifier un délimiteur généré par le dispositif de calcul avant la génération de la séquence d'étalonnage et/ou après la génération de l'indication.

7. Système (400) selon la revendication 2, dans lequel le capteur (422) est un capteur de lumière et l'indication est une indication visuelle, la séquence d'étalonnage et l'indication étant incluses dans une présentation visuelle générée sur une unité d'affichage (414) du dispositif de calcul.

8. Système (400) selon la revendication 7, dans lequel la séquence d'étalonnage inclut une séquence prédéterminée de couleurs et l'indication visuelle inclut une séquence d'images visuelles incluant une pluralité de couleurs, le processeur de stylo étant configuré pour reconnaître la séquence prédéterminée de couleurs incluse dans la séquence d'étalonnage, la séquence prédéterminée de couleurs de la séquence d'étalonnage incluant chaque couleur de la pluralité de couleurs incluse dans la séquence d'images visuelles de l'indication visuelle.

9. Procédé, comprenant :
la demande, via une interaction avec une interface utilisateur (414) d'un dispositif de calcul (410), de l'activation d'une cartouche (430) associée à une clé de cartouche et incluant un ensemble chauffant (434) et un processeur (432) de cartouche incluant la clé de cartouche stockée dans une mémoire pour un fonctionnement avec un stylo vaporisateur (420) incluant une alimentation électrique (424) configurée pour fournir une alimentation fonctionnelle à l'ensemble chauffant ;
la mise en prise de la cartouche avec le stylo vaporisateur ; et
la disposition du stylo vaporisateur adjacent au dispositif de calcul de sorte qu'un capteur (422) du stylo vaporisateur puisse détecter une séquence d'étalonnage et une indication générée par le dispositif de calcul, et qu'un processeur (425) du stylo vaporisateur puisse identifier un indicateur de clé en se basant sur la séquence d'étalonnage et l'indication détectée par le capteur, le processeur de cartouche (432) étant configuré pour comparer la clé de cartouche à l'indicateur de clé et déterminer s'il convient d'activer l'ensemble chauffant (434) en fonction du fait que l'indicateur de clé est basé ou non sur la clé de cartouche.

10. Procédé selon la revendication 9,
dans lequel l'indication inclut l'indicateur de clé, le stylo vaporisateur (420) étant configuré pour fournir une communication à la cartouche (430) en se basant sur l'indicateur de clé de sorte que la cartouche puisse déterminer si elle doit ou non permettre un fonctionnement de l'ensemble chauffant en fonction du fait que la communication est basée ou non sur l'indicateur de clé et en fonction du fait que l'indicateur de clé est associé ou non à une clé de cartouche stockée dans une mémoire de la cartouche.

11. Procédé selon la revendication 10,
dans lequel la communication est basée en partie sur une valeur de défi fournie au stylo vaporisateur (420) par la cartouche (430), et la cartouche peut déterminer si elle doit ou non autoriser le fonctionnement de l'ensemble chauffant en se basant sur le fait que la communication est basée ou non en partie sur la valeur de défi.

12. Procédé selon la revendication 9,
dans lequel la demande inclut au moins une action, par le dispositif de calcul (410), parmi le balayage d'un premier identifiant associé à la cartouche (430) ou la saisie manuelle d'un deuxième identifiant associé à la cartouche via une interaction avec l'interface utilisateur de sorte que le dispositif de calcul transmette le premier identifiant et/ou le deuxième identifiant à un centre de contrôle, l'indication générée par le dispositif de calcul étant basée sur des instructions envoyées au dispositif de calcul depuis le centre de contrôle en se basant sur au moins l'un du premier identifiant ou du deuxième identifiant.

13. Procédé selon la revendication 9,
dans lequel l'interface utilisateur inclut une unité d'affichage (414), l'indication est une indication visuelle, le capteur (422) est un capteur de lumière, la séquence d'étalonnage et l'indication visuelle incluant chacune une séquence d'images visuelles incluant une pluralité de couleurs, le stylo vaporisateur (420) est configuré pour reconnaître une séquence prédéterminée de couleurs incluse dans la séquence d'étalonnage, et la disposition inclut le placement d'une surface plane du stylo vaporisateur incluant une ouverture à l'intérieur de laquelle le capteur est disposé en contact avec l'unité d'affichage (414) du dispositif de calcul (410) de sorte que le capteur soit aligné avec l'unité d'affichage, chaque image visuelle de la séquence d'images visuelles affichées sur l'unité d'affichage du dispositif de calcul présentant une surface plus grande qu'une zone de surface de la surface plane du stylo vaporisateur en contact avec l'unité d'affichage.
